(19) Europäisches Patentamt / European Patent Office / Office européen des brevets

(11) **EP 4 473 912 B1**

(12) **EUROPEAN PATENT SPECIFICATION**

(45) Date of publication and mention
of the grant of the patent:
**03.06.2026 Bulletin 2026/23**

(21) Application number: **22931043.8**

(22) Date of filing: **15.12.2022**

(51) International Patent Classification (IPC):
*A61B 6/02* (2006.01)    *G06T 1/00* (2006.01)
*G06T 7/00* (2017.01)

(52) Cooperative Patent Classification (CPC):
**A61B 6/025; A61B 6/502; A61B 6/5205;**
**A61B 6/5264; G06T 7/00; G06T 7/0012;**
A61B 6/0414; G06T 2211/436

(86) International application number:
**PCT/JP2022/046280**

(87) International publication number:
**WO 2023/171073 (14.09.2023 Gazette 2023/37)**

(54) **IMAGE PROCESSING DEVICE, METHOD, AND PROGRAM**

BILDVERARBEITUNGSVORRICHTUNG, -VERFAHREN UND -PROGRAMM

DISPOSITIF, PROCÉDÉ ET PROGRAMME DE TRAITEMENT D'IMAGES

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **08.03.2022 JP 2022035379**

(43) Date of publication of application:
**11.12.2024 Bulletin 2024/50**

(73) Proprietor: **FUJIFILM Corporation**
**Tokyo 106-8620 (JP)**

(72) Inventor: **MORITA, Junya**
**Ashigarakami-gun, Kanagawa 258-8538 (JP)**

(74) Representative: **Dehns Germany Partnerschaft**
**mbB**
**Theresienstraße 6-8**
**80333 München (DE)**

(56) References cited:
WO-A1-2016/099924     WO-A1-2020/067475
WO-A1-2020/067475     JP-A- 2012 512 669
JP-A- 2016 064 118     JP-A- 2016 064 119
JP-A- 2020 005 706     JP-A- 2020 048 991
JP-A- 2020 089 667     US-A1- 2020 178 926
US-B2- 11 154 257

- YOSHINAGA YUKIYASU ET AL: "Evaluation Method of Concentration Degree and Convergence Index Filter", MEDICAL IMAGING TECHNOLOGY, vol. 19, no. 3, 1 May 2001 (2001-05-01), pages 154 - 160, XP093091277, DOI: 10.11409/mit.19.154
- YOSHINAGA YUKIYASU, HIDEFUMI KOBATAKE: "Evaluation Method of Concentration Degree and Convergence Index Filter", MEDICAL IMAGING TECHNOLOGY, vol. 19, no. 3, 1 May 2001 (2001-05-01), pages 154 - 160, XP093091277, DOI: 10.11409/mit.19.154

**Description**

BACKGROUND

Technical Field

The present disclosure relates to an image processing apparatus, an image processing method, and a non-transitory storage medium storing an image processing program.

Related Art

In recent years, in a radiography apparatus using radiation such as X-rays or gamma-rays, in order to observe a diseased part in more detail, there has been proposed tomosynthesis imaging of performing imaging by moving a radiation source and irradiating a subject with radiation at a plurality of radiation source positions, and deriving tomographic images in which desired tomographic planes are highlighted by adding a plurality of projection images acquired by the imaging. In the tomosynthesis imaging, the plurality of projection images are acquired by moving the radiation source in parallel with a radiation detector or so as to draw a circular or an elliptical arc according to characteristics of an imaging apparatus and required tomographic images and imaging the subject at the plurality of radiation source positions, and the tomographic images are derived by reconfiguring the projection images using an inverse projection method such as a simple inverse projection method or a filtering inverse projection method.

By deriving such a tomographic image on a plurality of tomographic planes of the subject, it is possible to separate structures overlapping each other in a depth direction in which the tomographic planes are aligned. Therefore, it is possible to find a lesion which is unlikely to be detected in the two-dimensional image acquired by simple imaging in the related art. The simple imaging is an imaging method for acquiring one two-dimensional image, which is a transmission image of a subject, by emitting radiation to the subject once.

On the other hand, the tomosynthesis imaging also has a problem that the reconstructed tomographic image is blurred due to an influence by a body movement or the like of the subject due to a time difference of imaging at each of a plurality of radiation source positions. In a case where the tomographic image is blurred as described above, it is difficult to find a lesion such as minute calcification, which is useful for early detection of breast cancer, particularly in a case where the breast is a subject.

For this reason, a method of correcting body movement in the case of deriving a tomographic image from a projection image acquired by tomosynthesis imaging has been proposed. For example, WO2020/067475A proposes a method of detecting at least one feature point in a derived tomographic image, deriving a misregistration amount between a plurality of projection images based on a body movement of a subject by using, as a reference, the feature point in a corresponding tomographic plane corresponding to the tomographic image from which the feature point is detected, reconstructing the plurality of projection images by correcting the misregistration amount, and deriving a corrected tomographic image in which an influence of the body movement is corrected.

SUMMARY OF INVENTION

Technical Problem

In the method disclosed in WO2020/067475A, the feature point is detected from the tomographic image. However, the feature point is not always detected on the projection image. For example, even in a case where a feature point is present with high contrast in the tomographic image, the contrast of the feature point is low in the projection image, and as a result, in some cases, it is difficult to detect the feature point. In a case where it is difficult to detect the feature point in the projection image, the feature point on the tomographic image and the feature point on the projection image cannot be accurately associated with each other, and in that case, a body movement cannot be accurately corrected.

Solution to Problem

The present disclosure has been made in view of the above circumstances, and an object of the present disclosure is to provide an image processing apparatus, an image processing method, and a non-transitory storage medium storing an image processing program capable of acquiring a tomographic image with high image quality in which a body movement is accurately corrected.

According to the present disclosure, there is provided an image processing apparatus comprising: at least one processor, in which the processor is configured to: acquire a plurality of projection images that are generated by performing, by an imaging apparatus, tomosynthesis imaging of relatively moving a radiation source with respect to a detection surface of a detection unit and irradiating a subject with radiation at a plurality of radiation source positions due to movement of the radiation source, the plurality of projection images corresponding to the plurality of radiation source positions; derive a plurality of feature-structure projection images by extracting a specific structure from the plurality of projection images; derive a plurality of feature-structure tomographic images respectively for a plurality of tomographic planes of the subject by reconstructing the plurality of feature-structure projection images; detect at least one feature structure from the plurality of feature-structure tomographic images; and derive a corrected tomographic image for at least one tomographic plane of the subject by correcting misregistration between the plurality of projection images due to a body movement of the subject by using, as a reference, the feature structure in a corresponding tomographic plane corresponding to the feature-structure tomographic image from which the feature structure is detected, and reconstructing the plurality of projection images.

The "relatively moving a radiation source with respect to a detection unit" includes a case of moving only the radiation source, a case of moving only the detection unit, and a case of moving both the radiation source and the detection unit.

In the image processing apparatus according to the present disclosure, the specific structure may be at least one of a line structure or a point structure.

Further, in the image processing apparatus according to the present disclosure, the processor may be configured to extract at least one of the line structure or the point structure based on a concentration degree of a gradient vector representing a gradient of pixel values in the projection image.

Further, in the image processing apparatus according to the present disclosure, the processor may be configured to: derive, in the corresponding tomographic plane, a misregistration amount between the plurality of projection images due to the body movement of the subject by using, as a reference, the feature structure; and derive the corrected tomographic image by correcting the misregistration amount and reconstructing the plurality of projection images.

Further, in the image processing apparatus according to the present disclosure, the processor may be configured to: detect a plurality of feature structures from the plurality of feature-structure tomographic images; determine whether or not the corresponding tomographic plane corresponding to the feature-structure tomographic image from which each of the plurality of feature structures is detected is a focal plane; and derive the misregistration amount in the corresponding tomographic plane determined as the focal plane.

Further, in the image processing apparatus according to the present disclosure, the processor may be configured to detect, as the feature structure, a point at which a specific threshold value condition is satisfied in the feature-structure tomographic image.

Further, in the image processing apparatus according to the present disclosure, the processor may be configured to: update the feature-structure tomographic image by reconstructing the feature-structure projection images while correcting the misregistration; detect an updated feature structure from the updated feature-structure tomographic image; update the misregistration amount using the updated feature structure; and repeat the update of the feature-structure tomographic image, the update of the feature structure, and the update of the misregistration amount.

Further, in the image processing apparatus according to the present disclosure, the processor may be configured to: update the feature-structure tomographic image by reconstructing the feature-structure projection images while correcting the misregistration; detect an updated feature structure from the updated feature-structure tomographic image based on an updated threshold value condition; update the misregistration amount by using the updated feature structure; and repeat the update of the feature-structure tomographic image, the update of the feature structure based on the updated threshold value condition, and the update of the misregistration amount.

Further, in the image processing apparatus according to the present disclosure, the processor may be configured to: derive a tomographic-plane projection image corresponding to each of the plurality of projection images by projecting the plurality of projection images onto the corresponding tomographic plane based on a positional relationship between the radiation source position and the detection unit when performing imaging for each of the plurality of projection images; and derive, in the corresponding tomographic plane, as the misregistration amount between the plurality of projection images, a misregistration amount between a plurality of the tomographic-plane projection images based on the body movement of the subject, by using, as a reference, the feature structure.

Further, in the image processing apparatus according to the present disclosure, the processor may be configured to: set a local region corresponding to the feature structure in the plurality of tomographic-plane projection images; and derive the misregistration amount based on the local region.

The term "local region" is a region including the feature structure in the tomographic image or the tomographic-plane projection image, and can be a region having any size smaller than the tomographic image or the tomographic-plane projection image.

The local region needs to be larger than a range of movement as the body movement. The body movement may be approximately 2 mm in a case of being large. Therefore, in a case of the tomographic image or the tomographic-plane projection image in which the size of one pixel is 100 $\mu$m square, the local region may be set to, for example, a region of 50 $\times$ 50 pixels or 100 $\times$ 100 pixels around the feature structure.

The term "region around the feature structure in the local region" means a region that is smaller than the local region and includes the feature structure in the local region.

Further, in the image processing apparatus according to the present disclosure, the processor may be configured to: set a plurality of first local regions including the feature structure in the plurality of tomographic-plane projection images; set a second local region including the feature structure in a tomographic image from which the feature structure is detected; derive misregistration amounts of the plurality of first local regions with respect to the second local region, as temporary misregistration amounts; and derive the misregistration amount based on a plurality of the temporary misregistration amounts.

Further, in the image processing apparatus according to the present disclosure, the processor may be configured to derive the temporary misregistration amounts based on a region around the feature structure in the second local region.

Further, in the image processing apparatus according to the present disclosure, the processor may be configured to: derive a plurality of the tomographic images as target tomographic images by reconstructing the plurality of projection images excluding a target projection image corresponding to a target tomographic-plane projection image that is a target for deriving the misregistration amount; and derive the misregistration amount for the target tomographic-plane projection image by using the target tomographic image.

Further, in the image processing apparatus according to the present disclosure, the processor may be configured to: perform image quality evaluation of a region of interest including the feature structure in the corrected tomographic image; and determine whether the derived misregistration amount is appropriate or inappropriate based on a result of the image quality evaluation.

Further, in the image processing apparatus according to the present disclosure, the processor may be configured to: derive a plurality of tomographic images by reconstructing the plurality of projection images; and perform image quality evaluation of a region of interest including the feature structure in the tomographic image; compare the result of the image quality evaluation for the corrected tomographic image with a result of the image quality evaluation for the tomographic image; and determine a tomographic image of which the result of the image quality evaluation is better as a final tomographic image.

Further, in the image processing apparatus according to the present disclosure, the processor may be configured to: derive an evaluation function for performing image quality evaluation of a region of interest including the feature structure in the corrected tomographic image; and derive the misregistration amount for optimizing the evaluation function.

Further, in the image processing apparatus according to the present disclosure, the subject may be a breast.

Further, in the image processing apparatus according to the present disclosure, the processor may be configured to change a search range in derivation of a misregistration amount according to at least one of a density of a mammary gland, a size of the breast, an imaging time of the tomosynthesis imaging, a compression pressure of the breast in the tomosynthesis imaging, or an imaging direction of the breast.

According to the present disclosure, there is provided an image processing method comprising: acquiring a plurality of projection images that are generated by performing, by an imaging apparatus, tomosynthesis imaging by relatively moving a radiation source with respect to a detection surface of a detection unit and irradiating a subject with radiation at a plurality of radiation source positions due to movement of the radiation source, the plurality of projection images corresponding to the plurality of radiation source positions; deriving a plurality of feature-structure projection images by extracting a specific structure from the plurality of projection images; deriving a plurality of feature-structure tomographic images respectively for a plurality of tomographic planes of the subject by reconstructing the plurality of feature-structure projection images; detecting at least one feature structure from the plurality of feature-structure tomographic images; and deriving a corrected tomographic image for at least one tomographic plane of the subject by correcting misregistration between the plurality of projection images based on a body movement of the subject by using, as a reference, the feature structure in a corresponding tomographic plane corresponding to the feature-structure tomographic image from which the feature structure is detected, and reconstructing the plurality of projection images.

According to the present disclosure, there is provided a non-transitory storage medium storing a program causing a computer to execute an image processing, the image processing comprising: acquiring a plurality of projection images that are generated by performing, by an imaging apparatus, tomosynthesis imaging by relatively moving a radiation source with respect to a detection surface of a detection unit and irradiating a subject with radiation at a plurality of radiation source positions due to movement of the radiation source, the plurality of projection images corresponding to the plurality of radiation source positions; deriving a plurality of feature-structure projection images by extracting a specific structure from the plurality of projection images; deriving a plurality of feature-structure tomographic images respectively for a plurality of tomographic planes of the subject by reconstructing the plurality of feature-structure projection images; detecting at least one feature structure from the plurality of feature-structure tomographic images; and deriving a corrected tomographic image for at least one tomographic plane of the subject by correcting misregistration between the plurality of projection images due to a body movement of the subject by using, as a reference, the feature structure in a corresponding tomographic plane corresponding to the feature-structure tomographic image from which the feature structure is detected, and reconstructing the plurality of projection images.

Effects of Invention

According to the present disclosure, it is possible to acquire a high-quality tomographic image in which the body movement is accurately corrected.

BRIEF DESCRIPTION OF THE DRAWINGS

Fig. 1 is a schematic configuration diagram of a radiography apparatus to which an image processing apparatus according to a first embodiment of the present disclosure is applied.

Fig. 2 is a diagram illustrating the radiography apparatus as viewed from a direction of an arrow A in Fig. 1.

Fig. 3 is a diagram illustrating a schematic configuration of the image processing apparatus according to the first embodiment.

Fig. 4 is a diagram illustrating a functional configuration of the image processing apparatus according to the first embodiment.

Fig. 5 is a diagram for describing acquisition of projection images.

Fig. 6 is a diagram illustrating a projection image and a feature-structure projection image.

Fig. 7 is a diagram for describing derivation of a feature-structure tomographic image.

Fig. 8 is a diagram for describing detection of a feature structure from the feature-structure tomographic image.

Fig. 9 is a diagram for describing projection of a projection image onto a corresponding tomographic plane.

Fig. 10 is a diagram for describing interpolation of pixel values of the tomographic image.

Fig. 11 is a diagram for describing setting of a region of interest.

Fig. 12 is a diagram illustrating a region of interest that is set in a tomographic-plane projection image.

Fig. 13 is a diagram illustrating an image in a region of interest in a case where no body movement occurs in the first embodiment.

Fig. 14 is a diagram illustrating an image in a region of interest in a case where body movement occurs in the first embodiment.

Fig. 15 is a diagram for describing a search range of the region of interest.

Fig. 16 is a diagram illustrating a feature structure in a three-dimensional space.

Fig. 17 is a diagram illustrating a display screen for a corrected tomographic image.

Fig. 18 is a flowchart illustrating processing performed in the first embodiment.

Fig. 19 is a diagram illustrating an image in a region of interest in a case where no body movement occurs in a second embodiment.

Fig. 20 is a diagram illustrating an image in a region of interest in a case where body movement occurs in the second embodiment.

Fig. 21 is a diagram for describing a region around a feature structure.

Fig. 22 is a diagram schematically illustrating processing performed in a third embodiment.

Fig. 23 is a flowchart illustrating processing performed in a fourth embodiment.

Fig. 24 is a diagram illustrating a warning display.

Fig. 25 is a diagram illustrating a functional configuration of the image processing apparatus according to a first embodiment.

Fig. 26 is a diagram for describing a ripple artifact.

Fig. 27 is a diagram for describing derivation of corresponding points.

Fig. 28 is a diagram illustrating a result obtained by plotting pixel values of feature structures and corresponding points.

Fig. 29 is a flowchart illustrating processing performed in a fifth embodiment.

Fig. 30 is a diagram illustrating a functional configuration of the image processing apparatus according to a sixth embodiment.

Fig. 31 is a diagram for describing setting of a region of interest in the sixth embodiment.

Fig. 32 is a flowchart illustrating processing performed in the sixth embodiment.

Fig. 33 is a diagram illustrating a functional configuration of the image processing apparatus according to a seventh embodiment.

DESCRIPTION OF EMBODIMENTS

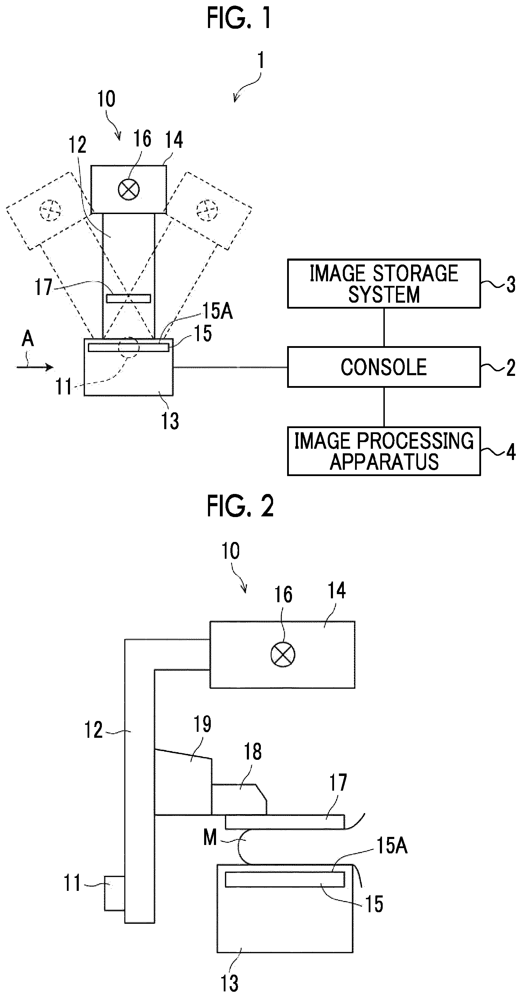

Hereinafter, embodiments of the present disclosure will be described with reference to the drawings. Fig. 1 is a schematic configuration diagram of a radiography system to which an image processing apparatus according to a first embodiment of the present disclosure is applied. As illustrated in Fig. 1, the radiography system 1 images a breast, which is a subject, at a plurality of radiation source positions and acquires a plurality of radiation images, that is, a plurality of projection images, in order to perform tomosynthesis imaging on the breast to derive tomographic images. The radiography system 1 comprises an imaging apparatus 10, a console 2, an image storage system 3, and an image processing apparatus 4 according to the present embodiment.

Fig. 2 is a diagram illustrating the imaging apparatus in the radiography system as viewed from a direction of an arrow A in Fig. 1. The imaging apparatus 10 is a mammography imaging apparatus that acquires a plurality of radiation images, that is, a plurality of projection images by imaging a breast M as a subject at a plurality of radiation source positions in order to derive a tomographic image by performing tomosynthesis imaging of the breast.

The imaging apparatus 10 comprises an arm part 12 that is connected to a base (not illustrated) by a rotation shaft 11. An imaging table 13 is attached to one end of the arm part 12, and a radiation irradiation unit 14 is attached to the other end of the arm part 12 so as to face the imaging table 13. The arm part 12 is configured such that only the end to which the radiation irradiation unit 14 is attached can be rotated. Therefore, the imaging table 13 is fixed and only the radiation irradiation unit 14 can be rotated. The rotation of the arm part 12 is controlled by the console 2.

A radiation detector 15, such as a flat panel detector, is provided in the imaging table 13. The radiation detector 15 has a detection surface 15A of radiation such as X-rays. In addition, a circuit board including a charge amplifier that converts a charge signal read from the radiation detector 15 into a voltage signal, a sampling two correlation pile circuit that samples the voltage signal output from the charge amplifier, and an analog-to-digital (AD) conversion unit that converts the voltage signal into a digital signal is provided in the imaging table 13. The radiation detector 15 is an example of a detection unit. Further, in the present embodiment, as the detection unit, the radiation detector 15 is used. On the other hand, the detection unit is not limited to the radiation detector 15 as long as the detection unit can detect radiation and convert the radiation into an image.

The radiation detector 15 can repeatedly perform recording and reading of a radiation image, may be a so-called direct-type radiation detector that directly converts radiation such as X-rays into charges, or may be a so-called indirect-type radiation detector that converts radiation into visible light once and converts the visible light into a charge signal. As a method for reading a radiation image signal, it is desirable to use the following method: a so-called thin film transistor (TFT) reading method which reads a radiation image signal by turning on and off a TFT switch; or a so-called optical reading method which reads a radiation image signal by irradiating a target with read light. On the other hand, the reading method is not limited thereto, and other methods may be used.

An X-ray source 16 that is a radiation source is accommodated in the radiation irradiation unit 14. The console 2 controls a timing when the X-ray source 16 emits an X-ray, which is radiation, and X-ray generation conditions of the X-ray source 16, that is, selection of a target and filter materials, a tube voltage, an irradiation time, and the like.

Further, the arm part 12 is provided with a compression plate 17 that is arranged above the imaging table 13 and presses and compresses the breast M, a support portion 18 that supports the compression plate 17, and a moving mechanism 19 that moves the support portion 18 in a vertical direction in Fig. 1 and Fig. 2.

The console 2 has a function of controlling the imaging apparatus 10 using, for example, an imaging order and various kinds of information acquired from a radiology information system (RIS) (not illustrated) or the like through a network, such as a wireless communication local area network (LAN), and commands or the like directly issued by an engineer or the like. Specifically, the console 2 acquires a plurality of projection images as described below by causing the imaging apparatus 10 to perform tomosynthesis imaging of the breast M. As an example, in the present embodiment, a server computer is used as the console 2.

The image storage system 3 is a system that stores image data such as a radiation image and a tomographic image which are obtained by imaging of the imaging apparatus 10. The image storage system 3 extracts image data corresponding to a request from the console 2 or the image processing apparatus 4 from the stored image data, and transmits the extracted image data to a device that is a source of the request. A specific example of the image storage system 3 is a picture archiving and communication system (PACS).

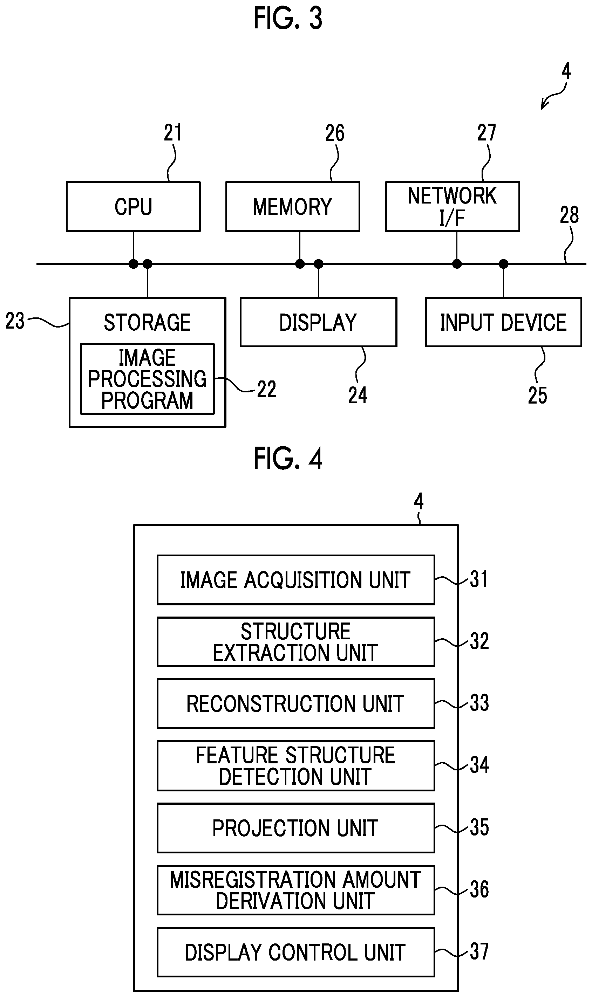

Next, the image processing apparatus according to the first embodiment will be described. Next, a hardware configuration of the image processing apparatus according to the first embodiment will be described with reference to Fig. 3. As illustrated in Fig. 3, the image processing apparatus 4 is a computer such as a workstation, a server computer, or a personal computer, and comprises a central processing unit (CPU) 21, a non-volatile storage 23, and a memory 26 as a temporary storage area. In addition, the image processing apparatus 4 comprises a display 24 such as a liquid crystal display, an input device 25 such as a keyboard and a mouse, and a network interface (I/F) 27 connected to a network (not illustrated). The CPU 21, the storage 23, the display 24, the input device 25, and the memory 26, and the network I/F 27 are connected to a bus 28. The CPU 21 is an example of a processor according to the present disclosure.

The storage 23 is implemented by a hard disk drive (HDD), a solid state drive (SSD), a flash memory, and the like. An image processing program 22 to be installed in the image processing apparatus 4 is stored in the storage 23 as a storage medium. The CPU 21 reads the image processing program 22 from the storage 23, expands the image processing program 22 in the memory 26, and executes the expanded image processing program 22.

The image processing program 22 is stored in a storage device of a server computer connected to the network or in a network storage in a state of being accessible from the outside, and is downloaded and installed in the computer that configures the image processing apparatus 4 in response to a request. Alternatively, the image processing program is distributed by being recorded on a recording medium such as a digital versatile disc (DVD) or a compact disc read only memory (CD-ROM), and is installed in a computer that configures the image processing apparatus 4 from the recording medium.

Next, a functional configuration of the image processing apparatus according to the first embodiment will be described. Fig. 4 is a diagram illustrating the functional configuration of the image processing apparatus according to the first embodiment. As illustrated in Fig. 4, the image processing apparatus 4 comprises an image acquisition unit 31, a structure extraction unit 32, a reconstruction unit 33, a feature structure detection unit 34, a projection unit 35, a misregistration amount derivation unit 36, and a display control unit 37. In addition, in a case where the CPU 21 executes the image processing program 22, the image processing apparatus 4 functions as the image acquisition unit 31, the structure extraction unit 32, the reconstruction unit 33, the feature structure detection unit 34, the projection unit 35, the misregistration amount derivation unit 36, and the display control unit 37.

The image acquisition unit 31 acquires a plurality of projection images generated in a case where the console 2 causes the imaging apparatus 10 to perform tomosynthesis imaging. The image acquisition unit 31 acquires a plurality of projection images from the console 2 or the image storage system 3 via the network I/F 27.

Here, the tomosynthesis imaging in the console 2 will be described. When performing the tomosynthesis imaging for generating a tomographic image, the console 2 moves the X-ray source 16 by rotating the arm part 12 around the rotation shaft 11. Further, the console 2 performs control to irradiate the breast M, which is the subject, with X-rays under predetermined imaging conditions for tomosynthesis imaging at a plurality of radiation source positions by the movement of the X-ray source 16. Further, the console 2 acquires a plurality of projection images Gi (i = 1 to n; n is the number of radiation source positions, for example, n = 15) at the plurality of radiation source positions which are obtained by detection of the X-rays transmitted through the breast M by the radiation detector 15.

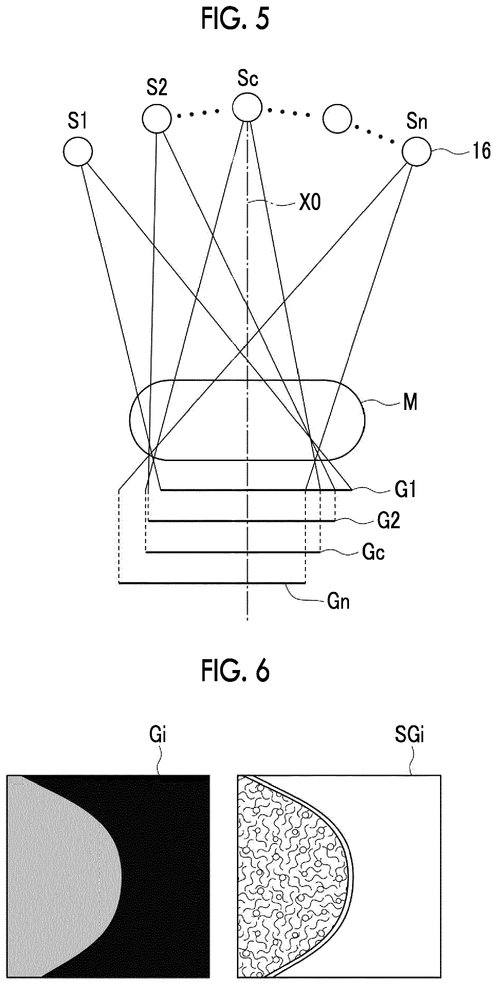

As illustrated in Fig. 5, the X-ray source 16 is moved to each of the radiation source positions Si (i = 1 to n), and the X-ray source 16 is driven to irradiate the breast M with X-rays at each of the radiation source positions. The radiation detector 15 detects the X-rays transmitted through the breast M, and thus the projection images G1, G2, ···, and Gn are acquired in correspondence with each of the radiation source positions S1 to Sn. At each of the radiation source positions S1 to Sn, the breast M is irradiated with X-rays having the same dose.

In Fig. 5, the radiation source position Sc is a radiation source position where an optical axis X0 of the X-rays emitted from the X-ray source 16 is perpendicular to the detection surface 15A of the radiation detector 15. In the following description, the radiation source position Sc is referred to as a reference radiation source position Sc, and the projection image Gc acquired by irradiating the breast M with X-rays at the reference radiation source position Sc is referred to as a reference projection image Gc. Here, "the optical axis X0 of the X-rays is perpendicular to the detection surface 15A of the radiation detector 15" means that the optical axis X0 of the X-rays crosses the detection surface 15A of the radiation detector 15 at an angle of 90°. On the other hand, the present disclosure is not limited thereto, and a case where the optical axis X0 of the X-rays crosses the detection surface 15A of the radiation detector 15 at an angle of 90° with a certain degree of error may be included. For example, a case where the optical axis X0 of the X-rays crosses the detection surface 15A of the radiation detector 15 at an angle of 90° with an error of approximately ±3° is also included in "the optical axis X0 of the X-rays is perpendicular to the detection surface 15A of the radiation detector 15" in the present embodiment.

The structure extraction unit 32 extracts a specific structure from each of the plurality of projection images Gi, and thus a plurality of feature-structure projection images are derived. Examples of the specific structure include at least one of a line structure or a point structure included in the breast M. Examples of the line structure include a mammary gland, a spicula, and a blood vessel. Examples of the point structure include calcification, an intersection of a plurality of mammary glands, and an intersection of blood vessels. The point structure is not limited to a fine point, and a region having a predetermined area is also included in the point structure. In addition, in the following description, it is assumed that both the line structure and the point structure are used as a specific structure. In order to extract the line structure and the point structure, in the present embodiment, a concentration degree of a gradient vector representing a gradient of pixel values of a projection image Gi, which is described in "Evaluation Method of Concentration Degree and Convergence Index Filter", Yoshinaga et al., Medical Imaging Technology, Vol. 19, No. 3, 2001", is used.

The method of extracting the line structure using the concentration degree is a method of obtaining a gradient vector on both sides of a search line in a certain direction of the projection image Gi by using a line concentration degree filter, evaluating a concentration degree at which the gradient vector is concentrated, and extracting a search line having a high evaluation value as the line structure. The method of extracting the point structure using the concentration degree is a method of obtaining a gradient vector in a certain direction of the projection image Gi by using a point concentration degree filter, evaluating a concentration degree at which the gradient vector is concentrated, and extracting a point having a high evaluation value as the point structure. By using the concentration degree of the vector, it is possible to extract the point structure or the line structure from the projection image Gi regardless of the contrast of the projection image Gi.

It is preferable to set a coefficient of the concentration degree filter such that a line structure having an actual dimension of approximately 1 mm in a case of a line structure and a point structure having an actual dimension of approximately 0.5 mm in a case of a point structure are extracted. Thereby, it is possible to prevent a noise included in the projection image Gi from being extracted as the line structure and the point structure.

In addition, the structure extraction unit 32 may extract an edge, an intersection of edges, a corner of edges, or the like included in the projection image Gi as the point structure by using an algorithm such as a Harris' corner detection method, scale-invariant feature transform (SIFT), features from accelerated segment test (FAST), or speeded up robust features (SURF).

Fig. 6 is a diagram illustrating a projection image and a feature-structure projection image. As illustrated in Fig. 6, the feature-structure projection image SGi is obtained by extracting the line structure included in the projection image Gi. The structure extraction unit 32 may derive a multi-valued feature-structure projection image SGi, or may derive a binary feature-structure projection image SGi by binarizing the multi-valued feature-structure projection image SGi.

In addition, the structure extraction unit 32 may extract at least one of the line structure or the point structure included in the projection image Gi by using a learning model obtained by machine learning.

The reconstruction unit 33 derives a feature-structure tomographic image in each of a plurality of tomographic planes of the breast M by reconstructing a plurality of feature-structure projection images SGi. In addition, the reconstruction unit 33 derives a tomographic image in which a desired tomographic plane of the breast M is emphasized by reconstructing all or some of the plurality of projection images Gi while correcting misregistration as described below. In addition, the reconstruction unit 33 derives a tomographic image in which a desired tomographic plane of the breast M is emphasized by reconstructing all or some of the plurality of projection images Gi without correcting a misregistration amount, as necessary.

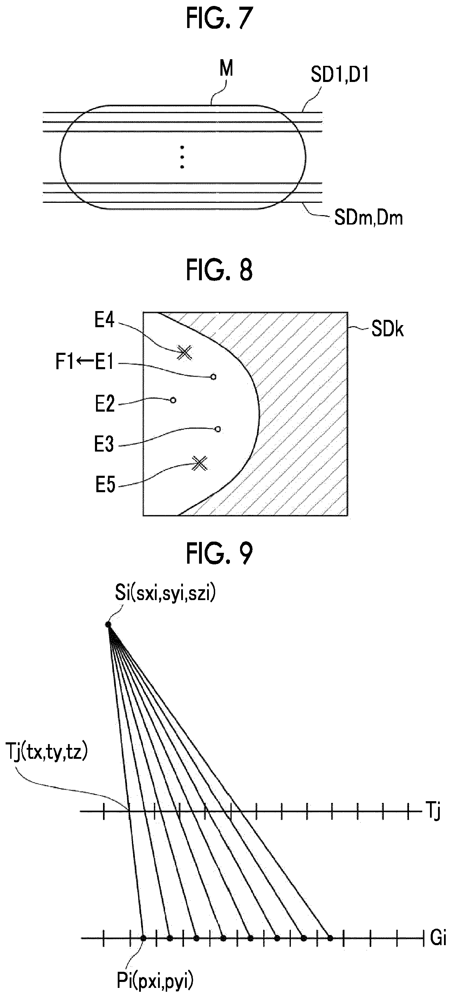

Specifically, the reconstruction unit 33 derives a plurality of feature-structure tomographic images SDj (j = 1 to m) in each of the plurality of tomographic planes of the breast M as illustrated in Fig. 7 by reconstructing the plurality of feature-structure projection images SGi using a well-known projection method such as a simple back projection method or a filter back projection method. Further, the reconstruction unit 33 derives a plurality of tomographic images Dj (j = 1 to m) in each of the plurality of tomographic planes of the breast M by reconstructing all or some of the plurality of projection images Gi. In this case, three-dimensional coordinate positions in a three-dimensional space including the breast M are set, and pixel values of corresponding pixel positions of the plurality of feature-structure projection images SGi or the plurality of projection images Gi are reconstructed with respect to the set three-dimensional coordinate positions. Thereby, the pixel values of the coordinate positions are calculated. In a case where a misregistration amount based on body movement of the breast M in the tomosynthesis imaging is derived as described later, the reconstruction unit 33 derives a corrected tomographic image in which the body movement is corrected by reconstructing the plurality of projection images Gi while correcting the misregistration.

The feature structure detection unit 34 detects at least one feature structure from a plurality of the feature-structure tomographic images SDj. Examples of the feature structure include a point structure and a line structure included in the feature-structure tomographic images SDj. Fig. 8 is a diagram for describing detection of the feature structure. In the present embodiment, it is assumed that the point structure is detected as the feature structure. In addition, here, detection of the feature structure from one feature-structure tomographic image SDk among the plurality of feature-structure tomographic images SDj will be described. As illustrated in Fig. 8, the feature-structure tomographic image SDk includes a point-shape structures E1 to E3, such as calcification, and intersections E4 and E5 of edges, such as intersections of blood vessels, in a tomographic plane of the breast M from which the feature-structure tomographic image SDk is acquired.

The feature structure detection unit 34 detects a point-shape structure such as calcification, as a point structure, that is, a feature structure, from the feature-structure tomographic image SDk by using a known algorithm. In addition, the feature structure detection unit 34 may detect, as a feature structure, a point such as an edge, an intersection of edges, and a corner of an edge included in the feature-structure tomographic image SDk by using an algorithm such as Harris' corner detection method, SIFT, FAST, or SURF. For example, the feature structure detection unit 34 detects the point-shape structure E1 included in the feature-structure tomographic image SDk illustrated in Fig. 8 as a feature structure F1. The point structure has a pixel value with high brightness, that is, a small pixel value in any of the feature-structure tomographic image SDk. Therefore, in any of these methods, the detected feature structure satisfies a specific threshold value condition. Specifically, the point structure is a point of which the pixel value is equal to or lower than a predetermined threshold value Th1 in the feature-structure tomographic image SDj. In a case where the pixel value of the feature-structure tomographic image SDj has a larger value as the brightness is higher, the detected feature structure is a point of which the pixel value is equal to or higher than the predetermined threshold value in the feature-structure tomographic image SDj.

As described above, the feature structure is not limited to the point structure. On the other hand, even in a case where a line structure is detected as the feature structure, for example, the brightness satisfying a specific threshold value condition can be used as a reference, or a known algorithm can be appropriately used. In addition, in order to detect the feature structure, an algorithm of computer aided diagnosis (CAD) may be used.

Here, for the sake of explanation, only one feature structure F1 is detected from one feature-structure tomographic image SDk. On the other hand, it is preferable to detect a plurality of feature structures. For example, all the point structures of the point-shape structures E1 to E3 and the intersections E4 and E5 included in the feature-structure tomographic image SDk illustrated in Fig. 8 may be detected as the feature structures. The feature structure may be only one pixel in the feature-structure tomographic image SDk, or may consist of a plurality of pixels indicating the position of the feature structure. In addition, here, for the sake of explanation, the feature structure is detected only from one feature-structure tomographic image SDk. On the other hand, actually, a plurality of feature structures are detected from each of a plurality of feature-structure tomographic images SDj.

The projection unit 35 projects the plurality of projection images Gi on the corresponding tomographic plane, which is the tomographic plane corresponding to the tomographic image from which the feature structure F1 is detected, based on the positional relationship between the radiation source position and the radiation detector 15 when performing imaging, for each of the plurality of projection images Gi. Thereby, the projection unit 35 derives tomographic-plane projection images GTi corresponding to each of the plurality of projection images Gi. Hereinafter, derivation of the tomographic-plane projection images GTi will be described. In the present embodiment, since the feature structure is detected in each of the plurality of tomographic images Dj, the plurality of projection images Gi are respectively projected on the plurality of tomographic planes Tj corresponding to the plurality of tomographic images Dj. Thereby, the tomographic-plane projection images GTi are derived.

Fig. 9 is a diagram for describing the projection of the projection images onto the corresponding tomographic planes. In Fig. 9, a case where one projection image Gi acquired at the radiation source position Si is projected onto one tomographic plane Tj of the breast M will be described. In the present embodiment, as illustrated in Fig. 9, at a position at which a straight line, which connects the radiation source position Si and the pixel position on the projection image Gi, and the tomographic plane Tj intersect with each other, the pixel value of the projection image Gi positioned on the straight line is projected.

The tomographic image derived from the projection image Gi and the tomographic plane Tj is an image consist of a plurality of pixels that are discretely arranged in a two-dimensional shape at a predetermined sampling interval, and is an image in which pixels are arranged at grid points having a predetermined sampling interval. In Fig. 9, a short line segment perpendicular to the projection image Gi and the tomographic plane Tj indicates a pixel division position. Therefore, in Fig.

9, the center position of the pixel division position is the pixel position which is at the grid point.

Here, a relationship of the coordinates (sxi, syi, szi) of the radiation source position at the radiation source position Si, the coordinates (pxi, pyi) of the pixel position Pi in the projection image Gi, and the coordinates (tx, ty, tz) of the projection position on the tomographic plane Tj is represented by the following Expression (1). In the present embodiment, a z-axis is set to a direction orthogonal to the detection surface 15A of the radiation detector 15, a y-axis is set to a direction parallel to a direction in which the X-ray source 16 moves on the detection surface of the radiation detector 15, and an x-axis is set to a direction perpendicular to the y-axis.

$$pxi = (tx \times szi - sxi \times tz)/(szi - tz)$$

$$pyi = (ty \times szi - syi \times tz)/(szi - tz) \cdots (1)$$

Therefore, by setting pxi and pyi in Expression (1) to the pixel position of the projection image Gi, and solving Expression (1) for tx and ty, the projection position on the tomographic plane Tj on which the pixel value of the projection image Gi is projected can be calculated. Therefore, by projecting the pixel value of the projection image Gi at the calculated projection position on the tomographic plane Tj, the tomographic-plane projection image GTi is derived.

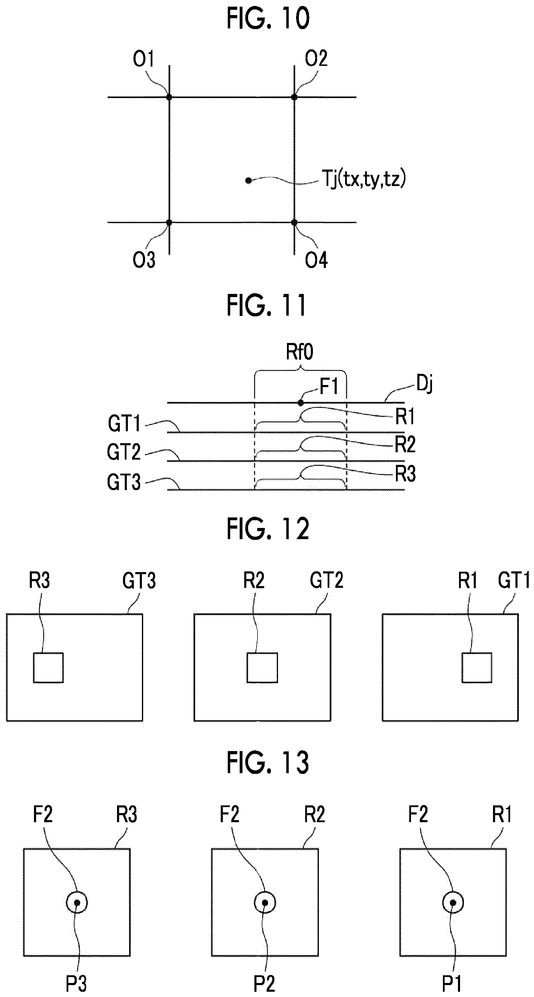

In this case, the intersection of the straight line, which connects the radiation source position Si and the pixel position on the projection image Gi, and the tomographic plane Tj may not be positioned on the pixel position on the tomographic plane Tj . For example, as illustrated in Fig. 10, the projection position (tx, ty, tz) on the tomographic plane Tj may be positioned between the pixel positions O1 to O4 of the tomographic image Dj on the tomographic plane Tj. In this case, the pixel value of each pixel position may be calculated by performing an interpolation calculation using the pixel value of the projection image at the plurality of projection positions around the pixel positions O1 to O4.

As the interpolation calculation, a linear interpolation calculation that weights the pixel value of the projection image at the projection position according to the distance between the pixel position and the plurality of projection positions around the pixel position can be used. In addition, any method such as a non-linear bicubic interpolation calculation using more pixel values of projection positions around the pixel position and a B-spline interpolation calculation can be used. Also, in addition to the interpolation calculation, the pixel value at the projection position closest to the pixel position may be used as the pixel value at the pixel position. Thereby, for the projection image Gi, the pixel values at all of the pixel positions of the tomographic plane Tj can be obtained. In the present embodiment, for each of the plurality of projection images Gi, the tomographic-plane projection image GTi having the pixel values obtained at all of the pixel positions of the tomographic plane Tj in this way is derived. Therefore, in one tomographic plane, the number of tomographic-plane projection images GTi matches with the number of projection images Gi.

The misregistration amount derivation unit 36 derives a misregistration amount between the plurality of tomographic-plane projection images GTi based on the body movement of the breast M during the tomosynthesis imaging. First, the misregistration amount derivation unit 36 sets a local region corresponding to the feature structure F1 as a region of interest for the plurality of tomographic-plane projection images GTi. Specifically, the local region having a predetermined size centered on the coordinate position of the feature structure F1 is set as the region of interest. Fig. 11 is a diagram for describing setting of the region of interest. In Fig. 11, for the sake of explanation, it is assumed that the tomographic-plane projection images GT1 to GT3 are derived by projecting three projection images G1 to G3 on the tomographic plane Tj.

As illustrated in Fig. 11, the misregistration amount derivation unit 36 sets the region of interest Rf0 centered on the coordinate position of the feature structure F1 in the tomographic image Dj on the tomographic plane Tj. The regions of interest R1 to R3 corresponding to the region of interest Rf0 are set in the tomographic-plane projection images GT1 to GT3. A broken line in Fig. 11 indicates a boundary between the regions of interest R1 to R3 and the other regions. Therefore, on the tomographic plane Tj, the positions of the region of interest Rf0 and the regions of interest R1 to R3 match with each other. Fig. 12 is a diagram illustrating the regions of interest R1 to R3 set in the tomographic-plane projection images GT1 to GT3. The body movement may be approximately 2 mm in a case of being large. Therefore, in a case of the tomographic image or the tomographic-plane projection image in which the size of one pixel is 100 $\mu$m square, the regions of interest R1 to R3 may be set to, for example, regions of 50 $\times$ 50 pixels or 100 $\times$ 100 pixels around the feature structure F1.

Further, the misregistration amount derivation unit 36 performs registration of the regions of interest R1 to R3. At this time, the registration is performed by using, as a reference, the region of interest that is set in the reference tomographic-plane projection image. In the present embodiment, the registration of other regions of interest is performed by using, as a reference, the region of interest that is set in the tomographic-plane projection image (reference tomographic-plane projection image) for the reference projection image (referred to as Gc) acquired at the radiation source position Sc at which the optical axis X0 of the X-rays from the X-ray source 16 is perpendicular to the radiation detector 15.

Here, it is assumed that the region of interest R2 illustrated in Fig. 12 is set in the reference tomographic-plane projection image. In this case, the misregistration amount derivation unit 36 performs registration of the regions of interest R1 and R3 with respect to the region of interest R2, and derives a shift vector representing the movement direction and the movement amount of the regions of interest R1 and R3 with respect to the region of interest R2, as a misregistration amount. The registration means that the movement direction and the movement amount of the regions of interest R1 and R3 with respect to the region of interest R2 are obtained in a predetermined search range such that the correlation between the regions of interest R1 and R3 and the region of interest R2 is maximized. Here, a normalized cross correlation may be used as the correlation. Further, since the one reference tomographic-plane projection image among the tomographic-plane projection images GTi is used as a reference, the shift vector is one less than the number of the tomographic-plane projection images. For example, in a case where the number of the tomographic-plane projection images is 15, the number of the shift vectors is 14. In a case where the number of the tomographic-plane projection images is 3, the number of the shift vectors is 2.

Fig. 13 is a diagram illustrating the images in three regions of interest R1 to R3 in a case where no body movement occurs during acquisition of the projection images G1 to G3. In Fig. 13, the center positions of the regions of interest R1 to R3, that is, the positions P1 to P3 corresponding to the feature structures F1 in the tomographic-plane projection images GT1 to GT3 are illustrated, and images F2 of the feature structures F1 included in the regions of interest R1 to R3 are indicated by a large circle. As illustrated in Fig. 13, in a case where no body movement occurs during the acquisition of the projection images G1 to G3, in all of three regions of interest R1 to R3, the positions P1 to P3 corresponding to the feature structures F1 and the positions of the images F2 of the feature structures F1 match with each other. Therefore, the shift vectors of the regions of interest R1 and R3 with respect to the region of interest R2, that is, the misregistration amounts are all 0.

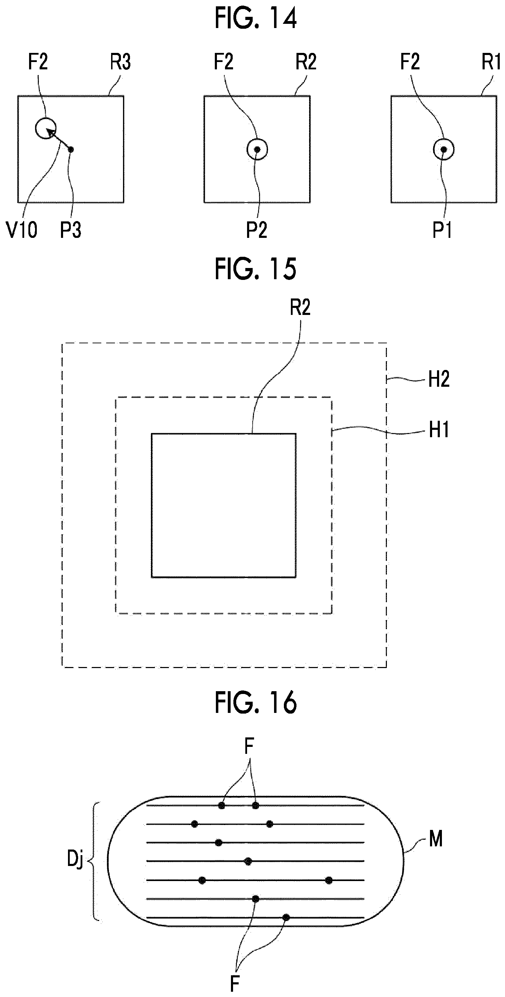

Fig. 14 is a diagram illustrating the images in three regions of interest R1 to R3 in a case where body movement occurs during acquisition of the projection image G2 and the projection image G3 among the projection images G1 to G3. In Fig. 14, since no body movement occurs during the acquisition of the projection image G1 and the projection image G2, the positions P1 and P2 corresponding to the feature structures F1 in the regions of interest R1 and R2 and the positions of the images F2 of the feature structures F1 included in the regions of interest R1 and R2 match with each other. For this reason, the misregistration amount of the region of interest R1 with respect to the region of interest R2 is 0. On the other hand, since no body movement occurs during the acquisition of the projection image G2 and the projection image G3, the position P3 corresponding to the feature structure F1 in the region of interest R3 and the position of the image F2 of the feature structure F1 included in the region of interest R3 do not match with each other. For this reason, due to the movement amount and the movement direction of the region of interest R3 with respect to the region of interest R2, the shift vector V10 having a magnitude and a direction is derived as the misregistration amount.

In a case where the misregistration amount is derived, a search range in a case of deriving the misregistration amount may be changed depending on at least one of a density of a mammary gland for the breast M, a size of the breast M, an imaging time of the tomosynthesis imaging, a compression pressure of the breast M in a case of the tomosynthesis imaging, or an imaging direction of the breast. Fig. 15 is a diagram for describing changing of the search range. As illustrated in Fig. 15, two types of search ranges, a narrow search range H1 and a wide search range H2, are set as the search ranges of the regions of interest R1 and R3 with respect to the region of interest R2 which is a reference.

Here, in a case where the density of the mammary gland is low, an amount of fat in the breast M is large, and thus the body movement tends to be large in a case of imaging. Also, in a case where the breast M is large, the body movement tends to be large in a case of imaging. In addition, as the tomosynthesis imaging time is longer, the body movement during imaging tends to be large. In addition, even in a case where the compression pressure of the breast M is low, the body movement tends to be large during imaging. Further, in a case where the imaging direction of the breast M is a medio-lateral oblique (MLO) direction, the body movement during imaging tends to be large than in a cranio-caudal (CC) direction.

Therefore, preferably, the misregistration amount derivation unit 36 changes a search range in a case of deriving the misregistration amount by receiving, from the input device 25, the input of at least one piece of information of a density of a mammary gland for the breast M, a size of the breast M, an imaging time of the tomosynthesis imaging, a compression pressure of the breast M in a case of the tomosynthesis imaging, or an imaging direction of the breast M. Specifically, in a case where the body movement tends to increase, the wide search range H2 illustrated in Fig. 15 may be set. On the contrary, in a case where the body movement tends to decreases, the narrow search range H1 illustrated in Fig. 15 may be set.

In the above, for the sake of explanation, the misregistration amount between the plurality of tomographic-plane projection images GTi is derived for one feature structure F1 on one tomographic plane Tj. In practice, however, as illustrated in Fig. 16, the misregistration amount derivation unit 36 derives misregistration amounts for a plurality of different feature structures F (here, ten feature structures illustrated by black circles) in a three-dimensional space of the breast M represented by the plurality of tomographic images Dj. As a result, for the tomographic-plane projection images corresponding to the projection images acquired in a state where the body movement occurs, the misregistration amounts for the plurality of different feature structures F are derived. The misregistration amount derivation unit 36 interpolates the misregistration amounts for the plurality of different feature structures F with respect to the coordinate positions in the three-dimensional space from which the tomographic images Dj are derived. Thereby, for the tomographic-plane projection images acquired in a state where the body movement occurs, the misregistration amount derivation unit 36 derives the misregistration amounts in a case of performing reconstruction for all of the coordinate positions in the three-dimensional space from which the tomographic images are derived.

In addition, the reconstruction unit 33 derives corrected tomographic image Dhj in which the body movement is corrected by reconstructing the projection image Gi while correcting the misregistration amount derived in this way. Specifically, in a case where the back projection method is used for reconstruction, the pixel of the projection image Gi in which the misregistration occurs is reconstructed by correcting the misregistration based on the derived misregistration amount such that the pixel corresponding to the other projection image is projected at the position for back projection.

Instead of deriving the misregistration amounts for the plurality of different feature structures F, one misregistration amount may be derived from the plurality of different feature structures F. In this case, the region of interest is set for each of the plurality of different feature structures F, and the misregistration amount is derived on an assumption that the entire region of interest moves in the same direction by the same amount. In this case, the misregistration amount may be derived such that a representative value (for example, an average value, a median value, or a maximum value) of the correlation for all of the regions of interest between the tomographic-plane projection images is maximized, the tomographic-plane projection images being targets of the derivation of the misregistration amount. Here, in a case where a signal-to-noise ratio of each feature structure F in the tomographic-plane projection image is not very good, the accuracy in the derivation of the misregistration amount deteriorates. On the other hand, by deriving one misregistration amount from the plurality of different feature structures F in this way, even in a case where the signal-to-noise ratio of each feature structure F is not very good, the accuracy in the derivation of the misregistration amount can be improved.

The three-dimensional space of the breast M represented by the plurality of tomographic images Dj may be divided into a plurality of three-dimensional regions, and one misregistration amount may be derived from the plurality of feature structures F in the same manner as described above for each region.

Further, in the present embodiment, the reconstruction unit 33 derives the plurality of tomographic images Dj by reconstructing the plurality of projection images Gi without correcting the misregistration amount.

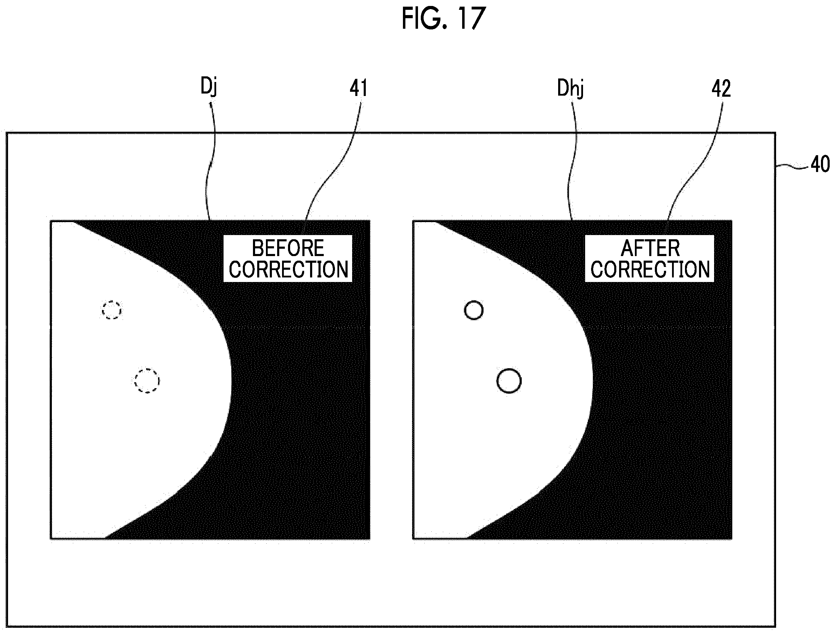

The display control unit 37 displays the corrected tomographic image that is derived on the display 24. Fig. 17 is a diagram illustrating a display screen for the corrected tomographic image. As illustrated in Fig. 17, the tomographic image Dj before body movement correction and the corrected tomographic image Dhj on which body movement correction is performed are displayed on a display screen 40. A label 41 of "before correction" is given to the tomographic image Dj such that it can be seen that the body movement is not corrected. A label 42 of "after correction" is given to the corrected tomographic image Dhj such that it can be seen that body movement is corrected. The label 41 may be given only to the tomographic image Dj, or the label 42 may be given only to the corrected tomographic image Dhj. It is needless to say that only the corrected tomographic image Dhj may be displayed. In Fig. 17, a broken line indicates that the structures included in the tomographic image Dj before correction is blurred, and a solid line indicates that the structures included in the corrected tomographic image Dhj is not blurred.

Further, it is preferable that the tomographic image Dj and the corrected tomographic image Dhj display the same cross section. In a case of switching the tomographic plane to be displayed according to an instruction from the input device 25, it is preferable to link the tomographic plane to be displayed in the tomographic image Dj and the corrected tomographic image Dhj. In addition to the tomographic image Dj and the corrected tomographic image Dhj, the projection image Gi may be displayed.

The operator can confirm the success or failure of the body movement correction by looking at the display screen 40. Further, in a case where the body movement is too large, even in a case where the tomographic image is derived by performing reconstruction while correcting the misregistration amount as in the present embodiment, the body movement cannot be corrected accurately, and the body movement correction may fail. In such a case, the tomographic image Dj may have a higher image quality than the corrected tomographic image Dhj due to the failure of the body movement correction. Therefore, the input device 25 may receive an instruction to store any of the tomographic image Dj or the corrected tomographic image Dhj, and the instructed image may be stored in the storage 23 or an external storage device.

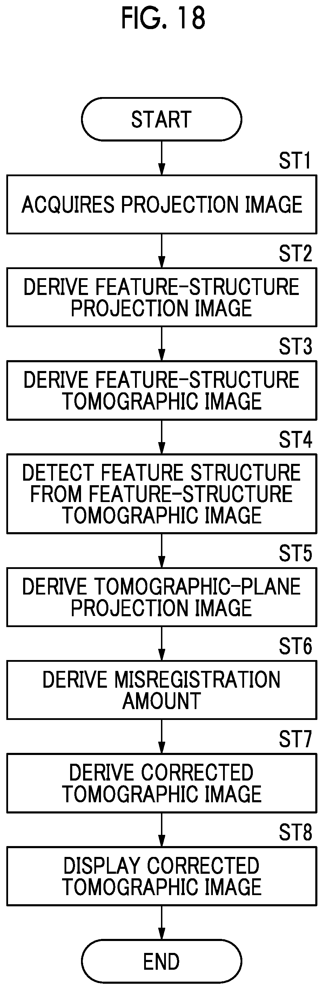

Next, processing performed in the first embodiment will be described. Fig. 18 is a flowchart illustrating processing performed in the first embodiment. In a case where the input device 25 receives a processing start instruction from the operator, the console 2 causes the imaging apparatus 10 to perform the tomosynthesis imaging on the breast M, and thus the image acquisition unit 31 acquires the plurality of projection images Gi derived by the tomosynthesis imaging (step ST1). In addition, the structure extraction unit 32 derives the plurality of feature-structure projection images SGi by extracting a specific structure from each of the plurality of projection images Gi (step ST2).

Subsequently, the reconstruction unit 33 derives the feature-structure tomographic images SDj in each of the plurality of tomographic planes of the breast M by reconstructing the plurality of feature-structure projection images SGi (step ST3). Next, the feature structure detection unit 34 detects at least one feature structure from a plurality of the feature-structure tomographic images SDj (step ST4). Further, the projection unit 35 projects the plurality of projection images Gi on the corresponding tomographic plane corresponding to the tomographic image from which the feature structure F1 is detected, based on the positional relationship between the radiation source position and the radiation detector 15 in a case of imaging each of the plurality of projection images Gi, and derives the tomographic-plane projection image GTi corresponding to each of the plurality of projection images Gi (step ST5).

Next, the misregistration amount derivation unit 36 derives the misregistration amount between the plurality of tomographic-plane projection images GTi (step ST6). Further, the reconstruction unit 33 derives the corrected tomographic image Dhj by reconstructing the plurality of projection images Gi while correcting the misregistration (step ST7). Moreover, the display control unit 37 displays the corrected tomographic image Dhj on the display 24 (step ST8), and the processing is ended. The corrected tomographic image Dhj that is derived is transmitted to the image storage system 3, and is stored in the image storage system 3.

Here, since a plurality of times of imaging is performed in a case where the tomosynthesis imaging is performed, the irradiation dose of the radiation in one imaging is small in order to reduce the exposure dose. As a result, the projection image Gi has a large amount of noise. For this reason, a structure such as calcification in the breast M may be buried in noise in the projection image Gi because the contrast of the structure is reduced depending on how the structures overlap with each other. Therefore, in a case where the feature structure is detected from the tomographic image derived by reconstructing the plurality of projection images Gi, it may not be possible to accurately associate the feature structure detected from the tomographic image with the structure corresponding to the feature structure included in the projection image. As a result, it may not be possible to accurately correct the misregistration of the projection image Gi using the feature structure.

In the first embodiment, the feature-structure projection image SGi is derived by extracting the specific structure, such as a line structure and a point structure, from the projection image, and the feature-structure tomographic image SDj is derived by reconstructing the feature-structure projection image SGi. The feature structure is detected from the feature-structure tomographic image SDj. Here, since the feature-structure tomographic image SDj is derived from the feature-structure projection image SGi, it is guaranteed that the structure corresponding to the feature structure detected from the feature-structure tomographic image SDj is included in the feature-structure projection image SGi and the projection image Gi. Therefore, according to the first embodiment, the misregistration amount between the plurality of projection images Gi can be appropriately derived by using the detected feature structure. As a result, according to the present embodiment, it is possible to acquire the high-quality corrected tomographic image Dhj in which the influence of the body movement is reduced.

Further, in the first embodiment, the feature structure is detected from a plurality of the feature-structure tomographic images SDj, instead of the projection image Gi or the tomographic-plane projection image GTi. Here, the feature-structure tomographic image SDj includes only the structure included in the corresponding tomographic plane Tj. For this reason, the structure on another tomographic plane included in the projection image Gi is not included in the feature-structure tomographic image SDj. Thus, according to the first embodiment, the feature structure can be accurately detected without being affected by the structures on other tomographic planes. Therefore, the misregistration amount between the plurality of projection images Gi can be appropriately derived. As a result, according to the present embodiment, the high-quality corrected tomographic image Dhj in which the influence of the body movement is reduced can be acquired.

Next, a second embodiment of the present disclosure will be described. A configuration of an image processing apparatus according to the second embodiment is the same as the configuration of the image processing apparatus according to the first embodiment illustrated in Fig. 4, and only the processing performed is different. Therefore, detailed description of the apparatus will be omitted here. In the first embodiment, the misregistration amount is derived between the tomographic-plane projection images GTi. In the second embodiment, the region of interest Rf0 centered on the coordinate position of the feature structure F1 is set in the feature-structure tomographic image SDj, and the misregistration amount of the region of interest Ri that is set in the tomographic-plane projection image GTi with respect to the set region of interest Rf0 is derived as a temporary misregistration amount. In addition, the difference from the first embodiment is that the misregistration amount between the plurality of tomographic-plane projection images GTi is derived based on the derived temporary misregistration amount. The region of interest Ri that is set in the plurality of tomographic-plane projection images GTi corresponds to a first local region, and the region of interest Rf0 that is set in the feature-structure tomographic image SDj corresponds to a second local region.

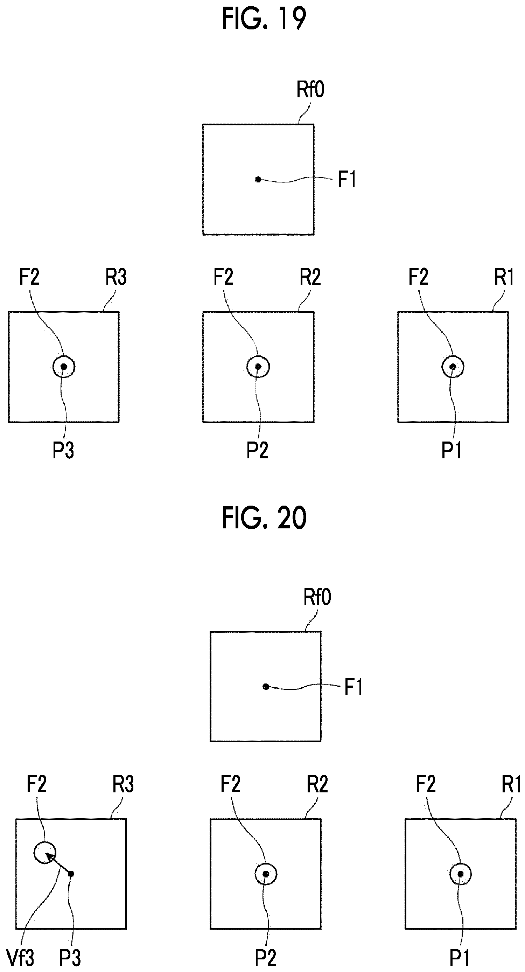

Fig. 19 is a diagram for describing derivation of the misregistration amount in the second embodiment. The region of interest Rf0 and the regions of interest R1 to R3 in Fig. 19 are the same as the region of interest Rf0 and the regions of interest R1 to R3 illustrated in Fig. 11 and the like. In the second embodiment, first, the misregistration amount derivation unit 36 derives, by using, as a reference, the region of interest Rf0 that is set in the feature-structure tomographic image SDj, the misregistration amounts of the regions of interest R1 to R3 that are set in the tomographic-plane projection images GTi (GT1 to GT3 in Fig. 19, (none of which are illustrated)) with respect to the region of interest Rf0, as temporary misregistration amounts. In a case where no body movement occurs during the acquisition of the projection images G1 to G3, in all of three regions of interest R1 to R3, the positions P1 to P3 corresponding to the feature structures F1 and the positions of the images F2 of the feature structures F1 match with each other. Therefore, shift vectors (hereinafter, referred to as Vfl, Vf2, and Vf3) of the regions of interest R1 to R3 with respect to the region of interest Rf0, that is, the temporary misregistration amounts, are all 0.

Fig. 20 is a diagram illustrating the images in three regions of interest R1 to R3 in a case where body movement occurs during acquisition of the projection image G2 and the projection image G3 among the projection images G1 to G3. In Fig. 20, since no body movement occurs during the acquisition of the projection image G1 and the projection image G2, the positions P1 and P2 corresponding to the feature structures F1 in the regions of interest R1 and R2 and the positions of the images F2 of the feature structures F1 included in the regions of interest R1 and R2 match with each other. For this reason, the misregistration amounts of the regions of interest R1 and R2 with respect to the region of interest Rf0 are 0. On the other hand, since no body movement occurs during the acquisition of the projection image G2 and the projection image G3, the position P3 corresponding to the feature structure F1 in the region of interest R3 and the position of the image F2 of the feature structure F1 included in the region of interest R3 do not match with each other. This leads to the movement amount and the movement direction of the region of interest R3 with respect to the region of interest Rf0. Therefore, the shift vectors Vf1 and Vf2 of the regions of interest R1 and R2 with respect to the region of interest Rf0, that is, the temporary misregistration amounts are 0, but the shift vector Vf3 of the region of interest R3 with respect to the region of interest Rf0, that is, the temporary misregistration amount has a value.

In the second embodiment, the misregistration amount derivation unit 36 derives the misregistration amount between the tomographic-plane projection images GTi based on the temporary misregistration amount. Specifically, as in the first embodiment, the misregistration amount is derived by using, as a reference, the projection image acquired at the reference radiation source position Sc at which the optical axis X0 of the X-rays from the X-ray source 16 is perpendicular to the radiation detector 15. Here, in a case where the projection image G2 is a reference tomographic-plane projection image, the misregistration amount derivation unit 36 derives the misregistration amount between the tomographic-plane projection image GT1 and the tomographic-plane projection image GT2 by a difference value Vf1 - Vf2 of the shift vectors Vf1 and Vf2 of the regions of interest R1 and R2 with respect to the region of interest Rf0. In addition, the misregistration amount derivation unit 36 derives the misregistration amount between the tomographic-plane projection image GT3 and the tomographic-plane projection image GT2 by a difference value Vf3 - Vf2 of the shift vectors Vf3 and Vf2 of the regions of interest R3 and R2 with respect to the region of interest Rf0.

As described above, in the second embodiment, the temporary misregistration amounts of the regions of interest R1 to R3 that are set in the tomographic-plane projection images GTi with respect to the region of interest Rf0 that is set in the feature-structure tomographic image SDj are derived, and the misregistration amount between the tomographic-plane projection images GTi is derived based on the temporary misregistration amounts. Here, since the region of interest Rf0 is set in the feature-structure tomographic image SDj, unlike the projection image Gi, only the structure on the tomographic plane from which the feature-structure tomographic image SDj is acquired is included. Therefore, according to the second embodiment, the influence of the structures included in the tomographic planes other than the tomographic plane in which the feature structure is set is reduced, and the misregistration amount is derived. Therefore, according to the second embodiment, the influence of the structures on other tomographic planes can be reduced, and thus the misregistration amount between the plurality of projection images Gi can be accurately derived. As a result, according to the second embodiment, a high-quality corrected tomographic image Dhj in which the influence of the body movement is reduced can be acquired.

In the second embodiment, as in the first embodiment, a search range in a case of deriving the misregistration amount may be changed depending on at least one of a density of a mammary gland for the breast M, a size of the breast M, an imaging time of the tomosynthesis imaging, a compression pressure of the breast M in a case of the tomosynthesis imaging, or an imaging direction of the breast M.

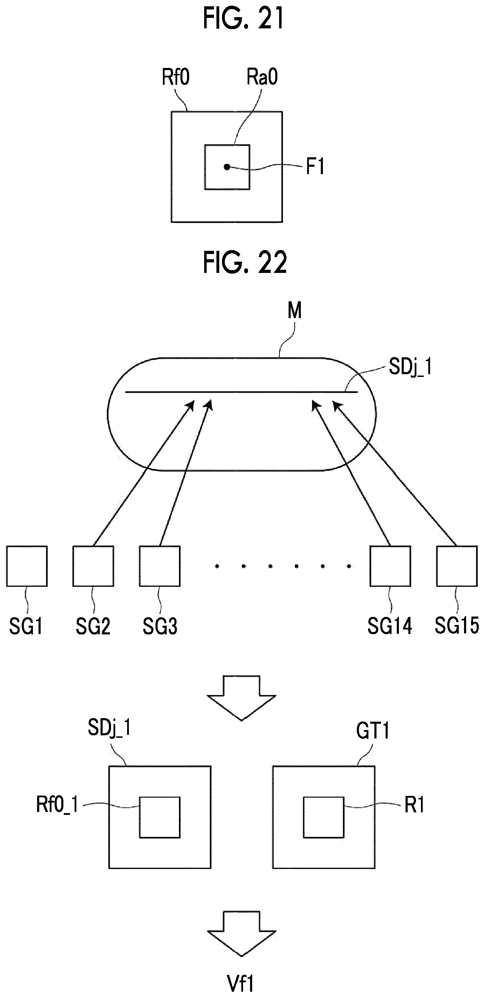

Further, in the second embodiment, the shift vectors Vf1 to Vf3 of the regions of interest R1 to R3 with respect to the region of interest Rf0 are derived as the temporary misregistration amounts. On the other hand, in this case, a peripheral region Ra0 that is smaller than the region of interest Rf0 may be set around the feature structure F1 of the region of interest Rf0 as illustrated in Fig. 21, and the shift vector may be derived based on the peripheral region Ra0. In this case, the shift vector may be derived using only the peripheral region Ra0. Further, in a case of deriving the correlation between the regions of interest R1 to R3, the peripheral region Ra0 may be weighted more heavily than the regions other than the peripheral regions Ra0 of the regions of interest R1 to R3.

Further, in the second embodiment, the region of interest Rf0 is set in the feature-structure tomographic image SDj. On the other hand, the feature-structure tomographic images to be derived may be different for each of the tomographic-plane projection images GTi for derivation of the temporary misregistration amount. Specifically, it is preferable to derive the feature-structure tomographic image excluding the target projection image corresponding to the target tomographic-plane projection image that is a target for derivation of the temporary misregistration amount. Hereinafter, this case will be described as a third embodiment.

Fig. 22 is a diagram schematically illustrating processing performed in a third embodiment. Here, a case of, assuming that, in the tomographic plane Tj of the breast M, among fifteen projection images G1 to G15, a projection image G1 is set as the target projection image and a tomographic-plane projection image GT1 is set as the target tomographic-plane projection image, deriving the temporary misregistration amount for the projection image G1 will be described. In this case, the reconstruction unit 33 derives a feature-structure tomographic image (SDj_1) by reconstructing the feature-structure projection images SG2 to SG15 other than the feature-structure projection image SG1 derived from the target projection image G1 in the tomographic plane Tj. In addition, the feature structure detection unit 34 detects the feature structure from the feature-structure tomographic image SDj_1, and the projection unit 35 derives the tomographic-plane projection images GT1 to GT15 from the projection images G1 to G15. The misregistration amount derivation unit 36 sets the region of interest Rf0_1 in the feature-structure tomographic image SDj_1, and derives the shift vector Vf1 of the region of interest R1 that is set in the tomographic-plane projection image GT1 with respect to the region of interest Rf0_1, as the temporary misregistration amount.

In a case where the temporary misregistration amount for the projection image G2 is derived, the reconstruction unit 33 derives a feature-structure tomographic image (referred to as SDj_2) by reconstructing the feature-structure projection images SG1, and SG3 to SG15 other than the feature-structure projection image SG2 derived from the projection image G2. In addition, the feature structure detection unit 34 detects the feature structure from the feature-structure tomographic image SDj_2, and the projection unit 35 derives the tomographic-plane projection images GT1 to GT15 from the projection images G1 to G15. The misregistration amount derivation unit 36 sets the region of interest Rf0_2 in the feature-structure tomographic image SDj_2, and derives the shift vector Vf2 of the region of interest R2 that is set in the tomographic-plane projection image GT2, as the temporary misregistration amount.

In addition, the temporary misregistration amounts for all of the tomographic-plane projection images GTi are derived by sequentially changing the target tomographic-plane projection image. As in the second embodiment, the misregistration amount between the tomographic-plane projection images GTi is derived based on the temporary misregistration amounts.

As described above, according to the third embodiment, the temporary misregistration amount is derived using the feature-structure tomographic image that is not affected by the target projection image. Therefore, the temporary misregistration amount can be more accurately derived, and as a result, the misregistration amount can be accurately derived.

In the third embodiment, in a case of reconstructing the feature-structure tomographic image excluding the feature-structure projection image for the target projection image, as shown in the following Expression (2), the feature-structure tomographic image may be derived by subtracting the corresponding pixel value Gp of the feature-structure projection image SGi derived from the target projection image Gi from the pixel value Dp of each pixel of the feature-structure tomographic image SDj derived by reconstructing all of the feature-structure projection images SGi and multiplying the subtracted pixel value by n/(n-1) times. Although the method of Expression (2) is a simple method, an amount of calculation for deriving the feature-structure tomographic image excluding the feature-structure projection image for the target projection image can be reduced. Therefore, it is possible to perform the processing for deriving the temporary misregistration amount at high speed.

$$\text{Feature-structure tomographic image excluding target projection image} = (Dp - Gp) \times n/(n - 1) \cdots (2)$$

Next, a fourth embodiment will be described. A configuration of the image processing apparatus according to the fourth embodiment is the same as the configuration of the image processing apparatus according to the first embodiment illustrated in Fig. 4, and only the processing performed is different. Therefore, detailed description of the device will be omitted here. The fourth embodiment is different from the first embodiment in that the feature-structure tomographic image SDj is updated by reconstructing the feature-structure projection images SGi while correcting the misregistration amount, that the updated feature structure is detected from the updated feature-structure tomographic image by using the updated threshold value, that the misregistration amount is updated by using the updated feature structure, and that the update of the feature-structure tomographic image, the detection of the updated feature structure by using the updated threshold value, and the update of the misregistration amount are repeated until the misregistration amount converges.

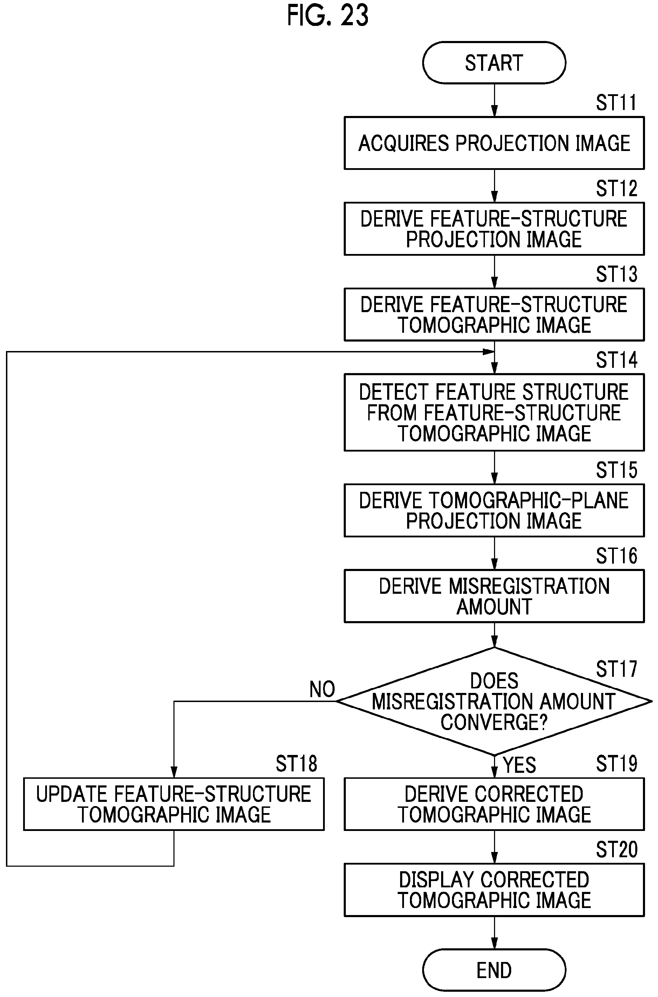

Fig. 23 is a flowchart illustrating processing performed in the fourth embodiment. In Fig. 23, the processing from step ST11 to step ST16 is the same as the processing from step ST1 to step ST6 illustrated in Fig. 18, and thus detailed description thereof will be omitted here. In a case where the misregistration amount is derived in step ST16, the misregistration amount derivation unit 36 determines whether or not the misregistration amount converges (step ST17). The determination as to whether or not the misregistration amount converges may be performed by determining whether or not the misregistration amount derived for each tomographic-plane projection image GTi is equal to or smaller than a predetermined threshold value Th10. The threshold value Th10 may be set to a value at which it can be said that there is no influence of the body movement on the tomographic image without correcting the misregistration amount any more. The determination as to whether or not the misregistration amount converges may be performed by determining whether or not a representative value such as an average value of the misregistration amounts derived for the plurality of tomographic-plane projection images GTi is equal to or smaller than a predetermined threshold value Th10.

In a case where a determination result in step ST17 is "No", the reconstruction unit 33 updates the feature-structure tomographic image by reconstructing the plurality of feature-structure projection images SGi while correcting the misregistration amount (step ST18). In addition, the process returns to the processing of step ST14, and processing of step ST14 to step ST17 is performed. In this case, in the processing of step ST14, the feature structure detection unit 34 updates the threshold value Th1 to be used in a case of detecting the feature structure in the first processing of step ST14. In the present embodiment, since the pixel value of the feature-structure tomographic image SDj has a smaller value as the brightness is higher, the updated feature structure is detected by using the updated threshold value Th2, which is smaller than the threshold value Th1 used in the first processing of step ST14.

In addition, in the processing of step ST15, the projection unit 35 projects the plurality of projection images Gi onto the corresponding tomographic plane corresponding to the tomographic image from which the updated feature structure F1 is detected, based on the positional relationship between the radiation source position and the radiation detector 15 when performing imaging for each of the plurality of projection images Gi, and derives the updated tomographic-plane projection images GTi corresponding to each of the plurality of projection images Gi. In addition, in the processing of step ST16, the misregistration amount derivation unit 36 derives the updated misregistration amount between the plurality of updated tomographic-plane projection images GTi.

In a case where the pixel value of the feature-structure tomographic image SDj has a larger value as the brightness is higher, the updated feature structure is detected by using the updated threshold value Th2, which is larger than the threshold value Th1 used in the first processing of step ST14.

In a case where a determination result in step ST17 is No, processing of step ST18 and step ST14 to step ST16 is repeated until the determination result in step ST17 is Yes. In this case, the feature structure detection unit 34 detects the feature structure from the updated feature-structure tomographic image SDj by using the updated threshold value.

In a case where the determination result in step ST17 is Yes, the reconstruction unit 33 derives the corrected tomographic images Dhj by reconstructing the plurality of projection images Gi while correcting the updated misregistration amount (step ST19). In addition, the display control unit 37 displays the corrected tomographic image Dhj on the display 24 (step ST20), and the processing is ended. The corrected tomographic image Dhj that is derived is transmitted to the image storage system 3, and is stored in the image storage system 3.

As described above, in the fourth embodiment, the feature-structure tomographic image SDj is updated by reconstructing the feature-structure projection images SGi while correcting the misregistration amount. The updated feature structure is detected from the updated feature-structure tomographic image by using the updated threshold value. The misregistration amount is updated by using the updated feature structure. The update of the feature-structure tomographic image, the detection of the updated feature structure by using the updated threshold value, and the update of the misregistration amount are repeated until the misregistration amount converges. Therefore, the misregistration due to the body movement can be removed more appropriately and efficiently, and thus, it is possible to acquire a higher-quality tomographic image.

Also in the second embodiment and the third embodiment, the processing of updating the misregistration amount may be repeated until the misregistration amount converges as in the fourth embodiment.

In addition, in the fourth embodiment, while updating the threshold value, the update of the feature-structure tomographic image, the detection of the updated feature structure by using the updated threshold value, and the update of the misregistration amount are repeated until the misregistration amount converges. On the other hand, the present disclosure is not limited thereto. The update of the feature-structure tomographic image, the detection of the updated feature structure, and the update of the misregistration amount may be repeated without updating the threshold value.

In addition, in the fourth embodiment, the processing of updating the misregistration amount is repeated until the misregistration amount converges. On the other hand, the present disclosure is not limited thereto. The processing of updating the misregistration amount may be repeated a predetermined number of times.

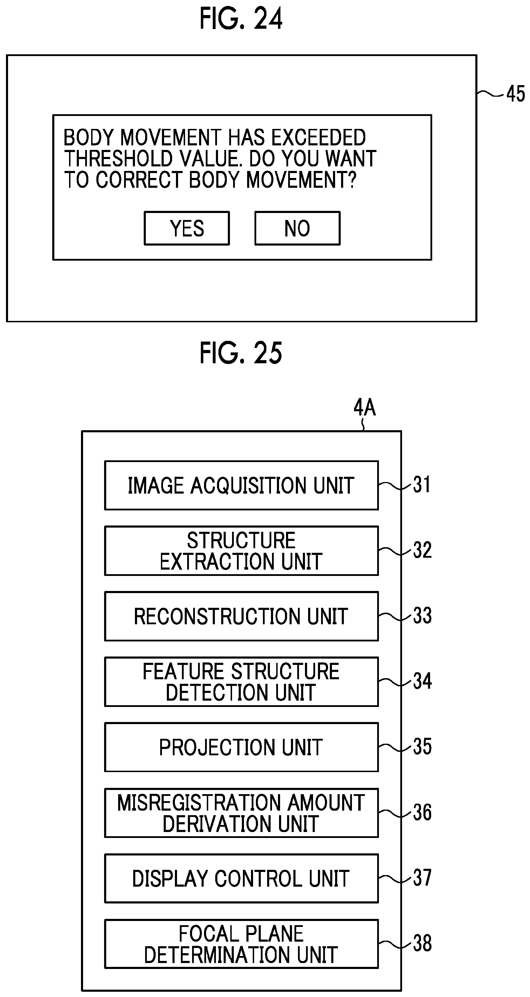

Further, in the above embodiments, the misregistration amount derived by the misregistration amount derivation unit 36 may compared with a predetermined threshold value, and only in a case where the misregistration amount exceeds the threshold value, the tomographic image may be reconstructed while correcting the misregistration amount. The threshold value may be set to a value at which it can be said that there is no influence of the body movement on the tomographic image without correcting the misregistration amount. In this case, as illustrated in Fig. 24, a warning display 45 for notifying that the body movement exceeds the threshold value may be displayed on the display 24. The operator can instruct whether to perform body movement correction by selecting YES or NO on the warning display 45.

In the above embodiments, in order to easily derive the misregistration amount and the temporary misregistration amount, the regions of interest are set in the feature-structure tomographic image SDj and the tomographic-plane projection image GTi, and the movement direction and the movement amount of the region of interest are derived as the shift vector, that is, the misregistration amount and the temporary misregistration amount. On the other hand, the present disclosure is not limited thereto. The misregistration amount may be derived without setting the region of interest.

Next, a fifth embodiment of the present disclosure will be described. Fig. 25 is a diagram illustrating a functional configuration of the image processing apparatus according to a fifth embodiment. In Fig. 25, the same reference numerals as those in Fig. 4 are assigned to the same configurations as those in Fig. 4, and detailed description thereof will be omitted here. The fifth embodiment is different from the first embodiment in that the image processing apparatus 4A further comprises a focal plane determination unit 38 that determines whether or not the corresponding tomographic plane corresponding to the feature-structure tomographic image from which each of the plurality of feature structures F is detected is a focal plane, and that the misregistration amount derivation unit 36 derives the misregistration amount in the corresponding tomographic plane determined as the focal plane. The processing according to the fifth embodiment can be applied to the second to fourth embodiments, but only a case where the processing is applied to the first embodiment will be described here.

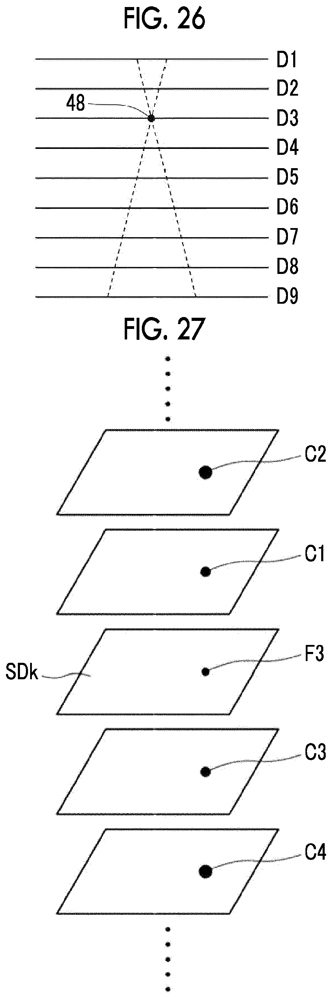

Here, in the tomographic image acquired by the tomosynthesis imaging, the reflected glare of the structure occurs in the tomographic image other than the tomographic image in which the structure is present. This is called a ripple artifact. Fig. 26 is a diagram for describing a ripple artifact. As illustrated in Fig. 26, assuming that a certain structure 48 is included in the tomographic image D3, the tomographic image corresponding to the upper and lower tomographic planes of the tomographic image D3 includes the ripple artifact of the structure 48. The ripple artifact becomes widely spread and more blurred as the distance from the tomographic plane including the structure 48 increases. The range in which the ripple artifact spreads corresponds to the range in which the X-ray source 16 moves. In addition, the ripple artifact also occurs in the feature-structure tomographic image SDj.

Here, in a case where the feature structure F detected by the feature structure detection unit 34 from the feature-structure tomographic image SDj of the corresponding tomographic plane is the ripple artifact, the feature structure F is blurred and widely spread. For this reason, in a case where such a feature structure F is used, the misregistration amount cannot be accurately derived.

Therefore, in the fifth embodiment, the focal plane determination unit 38 determines whether or not the corresponding tomographic plane for the feature-structure tomographic image SDj from which the feature structure F is detected is a focal plane, the projection unit 35 derives the tomographic-plane projection images GTi in the corresponding tomographic plane determined as a focal plane, and the misregistration amount derivation unit 36 derives the misregistration amount. Specifically, the misregistration amount is derived by using the feature structure detected from the feature-structure tomographic image in the corresponding tomographic plane determined as a focal plane. Hereinafter, the determination as to whether or not the corresponding tomographic plane is a focal plane will be described.

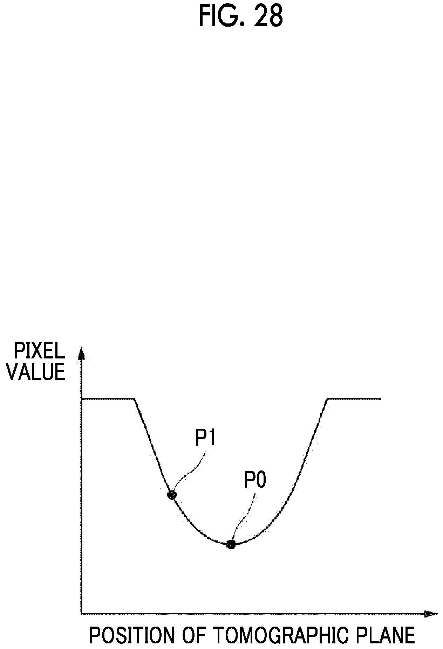

The focal plane determination unit 38 derives corresponding points corresponding to the feature structures in the plurality of feature-structure tomographic images SDj for the feature structures detected by the feature structure detection unit 34. Fig. 27 is a diagram for describing derivation of the corresponding points. As illustrated in Fig. 27, in a case where the feature structure F3 is detected in a certain feature-structure tomographic image SDk, the misregistration amount derivation unit 36 derives the corresponding points C1, C2, C3, C4, ... corresponding to the feature structure F3 in the plurality of feature-structure tomographic images positioned in a thickness direction of the feature-structure tomographic image SDk. In the following description, a reference numeral of the corresponding points is C. The corresponding points C may be derived by aligning the region of interest including the feature structure F3 with the feature-structure tomographic image other than the feature-structure tomographic image SDk. In addition, the focal plane determination unit 38 plots the pixel values of the feature structure F3 and the corresponding points C in an order in which the tomographic planes are arranged. Fig. 28 is a diagram showing a result obtained by plotting the pixel values of the feature structure and the corresponding points. As illustrated in Fig. 28, the pixel values of the feature structure and the corresponding points change to have a minimum value in the feature structure due to the influence of the ripple artifact. Here, in a case where the feature structure F3 is present in the focal plane, the feature structure F3 is not blurred and has high brightness, that is, a low pixel value. On the other hand, in a case where the feature structure F3 is not present in the focal plane, the feature structure F3 becomes a ripple artifact, and as a result, the pixel value is blurred and the pixel value becomes larger than the minimum value.

Therefore, in a case where the position of the tomographic plane in which the feature structure F3 is detected is the position P0 illustrated in Fig. 28 at which the pixel value is the minimum in the result obtained by plotting the pixel values of the feature structure F3 and the corresponding points, the focal plane determination unit 38 determines that the corresponding tomographic plane in which the feature structure F3 is detected is the focal plane. On the other hand, in a case where the position of the tomographic plane in which the feature structure F3 is detected is the position P1 or the like illustrated in Fig. 28 at which the pixel value is not the minimum, the focal plane determination unit 38 determines that the corresponding tomographic plane in which the feature structure F3 is detected is not the focal plane.

The projection unit 35 derives the tomographic-plane projection image GTi only in the corresponding tomographic plane determined as the focal plane, as in the above embodiments. The misregistration amount derivation unit 36 derives the misregistration amount of the tomographic-plane projection image GTi in the corresponding tomographic plane determined as the focal plane. That is, the misregistration amount derivation unit 36 derives the misregistration amount of the tomographic-plane projection image GTi by using the feature structure detected in the corresponding tomographic plane determined as the focal plane.

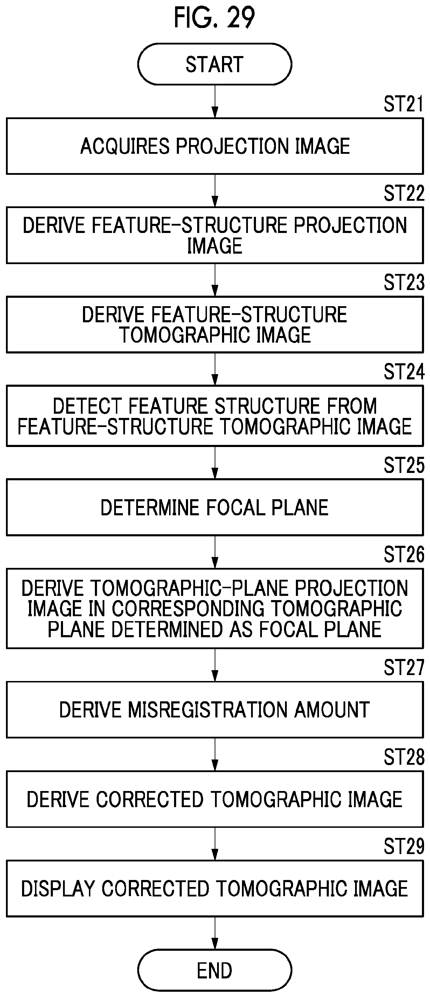

Next, processing performed in the fifth embodiment will be described. Fig. 29 is a flowchart illustrating processing performed in the fifth embodiment. In Fig. 29, the processing from step ST21 to step ST24 is the same as the processing from step ST1 to step ST4 illustrated in Fig. 18, and thus detailed description thereof will be omitted here. In the fifth embodiment, it is assumed that a plurality of feature structures are detected.

In a case where the feature structure detection unit 34 detects a plurality of feature structures, the focal plane determination unit 38 determines whether or not a corresponding tomographic plane, which corresponds to the feature-structure tomographic image from which each of the plurality of feature structures is detected by the feature structure detection unit 34, is the focal plane (focal plane determination; step ST25). In addition, the projection unit 35 derives the tomographic-plane projection image GTi in the corresponding tomographic plane determined as the focal plane (step ST26), and the misregistration amount derivation unit 36 derives the misregistration amount by using the feature structure detected in the feature-structure tomographic image of the corresponding tomographic plane determined as the focal plane (step ST27).

Further, the reconstruction unit 33 derives the corrected tomographic image Dhj by reconstructing the plurality of projection images Gi while correcting the misregistration amount (step ST28). In addition, the display control unit 37 displays the corrected tomographic image Dhj on the display 24 (step ST29), and the processing is ended. The corrected tomographic image Dhj that is derived is transmitted to the image storage system 3, and is stored in the image storage system 3.

As described above, in the fifth embodiment, the misregistration amount is derived in the corresponding tomographic plane determined as the focal plane. Therefore, the misregistration amount can be accurately derived without being affected by the ripple artifact, and as a result, the corrected tomographic image Dhj in which the misregistration is accurately corrected can be derived.

In the fifth embodiment, determination as to whether or not the corresponding tomographic plane is the focal plane by using the result obtained by plotting the pixel values of the feature structure and the corresponding points is performed. On the other hand, the determination as to whether or not the corresponding tomographic plane is the focal plane is not limited thereto. In the feature structure and the ripple artifact, a difference in contrast with surrounding pixels is larger in the feature structure than in the ripple artifact. Therefore, a difference in contrast with surrounding pixels in the feature structure and the corresponding points may be derived, and in a case where the contrast for the feature structure is the maximum, the corresponding tomographic plane in which the feature structure is detected may be determined as the focal plane. In addition, the pixel value at the position corresponding to the feature structure in the projection image has a small variation between the projection images in a case where the feature structure is present in the focal plane. On the other hand, in a case where the feature structure is not present in the focal plane, the pixel value at the position corresponding to the feature structure in the projection image may represent a structure other than the structure corresponding to the feature structure, and as a result, the pixel value has a large variation between the projection images. Therefore, a variance value of the pixel value corresponding to the feature structure between the projection images Gi may be derived, and in a case where the variance value is equal to or smaller than a predetermined threshold value, the corresponding tomographic plane in which the feature structure is detected may be determined as the focal plane. Further, the focal plane determination unit 38 may include a discriminator that is obtained by machine learning, receives the feature structure and the pixel value in the periphery of the feature structure, and outputs a determination result as to whether or not the corresponding tomographic plane in which the feature structure is detected is the focal plane. In this case, the discriminator may determine whether or not the corresponding tomographic plane in which the feature structure is detected is the focal plane.

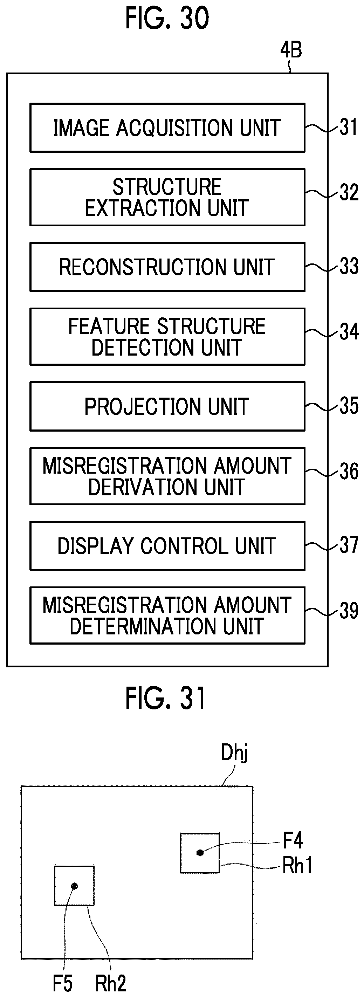

Next, a sixth embodiment of the present disclosure will be described. Fig. 30 is a diagram illustrating a functional configuration of the image processing apparatus according to a sixth embodiment. In Fig. 30, the same reference numerals as those in Fig. 4 are assigned to the same configurations as those in Fig. 4, and detailed description thereof will be omitted here. The sixth embodiment is different from the first embodiment in that the image processing apparatus 4B further comprises a misregistration amount determination unit 39 that performs image quality evaluation for a region of interest including the feature structure in the corrected tomographic image Dhj and determines whether or not the derived misregistration amount is appropriate or inappropriate based on a result of the image quality evaluation. The processing according to the sixth embodiment can be applied to the second to fifth embodiments, but only a case where the processing is applied to the first embodiment will be described here.

The misregistration amount determination unit 39 sets, for the image quality evaluation, the regions of interest Rh1 and Rh2 centered on the coordinate positions of the plurality (here, two) of the feature structures F4 and F5 included in the corrected tomographic image Dhj illustrated in Fig. 31. In addition, a high-frequency image is derived by extracting high-frequency components in each of the regions of interest Rh1 and Rh2. The extraction of the high-frequency components may be performed by performing filtering processing using a Laplacian filter and deriving a secondary differential image. On the other hand, the present disclosure is not limited thereto. Further, the misregistration amount determination unit 39 derives the magnitudes of the high-frequency components of the regions of interest Rh1 and Rh2. The magnitudes of the high-frequency components may be derived by the sum of squares of the pixel value of the high-frequency image. On the other hand, the present disclosure is not limited thereto. In addition, the misregistration amount

**EP 4 473 912 B1**

determination unit 39 derives the sum of the magnitudes of the high-frequency components of all of the regions of interest Rh1 and Rh2.

In a case where the misregistration correction is appropriately performed by deriving the misregistration amount appropriately, the image blurriness of the corrected tomographic image Dhj decreases, and the high-frequency components increase. On the other hand, in a case where the misregistration correction is inappropriate because the derived misregistration amount is not appropriate, the image blurriness of the corrected tomographic image Dhj increases, and the high-frequency components decrease. Therefore, in the sixth embodiment, the misregistration amount determination unit 39 performs the image quality evaluation based on the magnitudes of the high-frequency components. That is, the misregistration amount determination unit 39 determines whether or not the sum of the magnitudes of the high-frequency components of all of the regions of interest Rh1 and Rh2, which are derived as above, is equal to or larger than a predetermined threshold value Th20. In a case where the sum is equal to or larger than the threshold value Th20, the misregistration amount determination unit 39 determines that the misregistration amount is appropriate, and in a case where the sum is smaller than the threshold value Th20, the misregistration amount determination unit 39 determines that the misregistration amount is inappropriate. In a case where the misregistration amount determination unit 39 determines that the misregistration amount is inappropriate, the reconstruction unit 33 derives the tomographic images Dj by reconstructing the plurality of projection images Gi without correcting the misregistration amount. In addition, the display control unit 37 displays the tomographic image Dj before correction on the display 24 instead of the corrected tomographic image Dhj. In this case, instead of the corrected tomographic image Dhj, the tomographic image Dj before correction is transmitted to an external storage device.

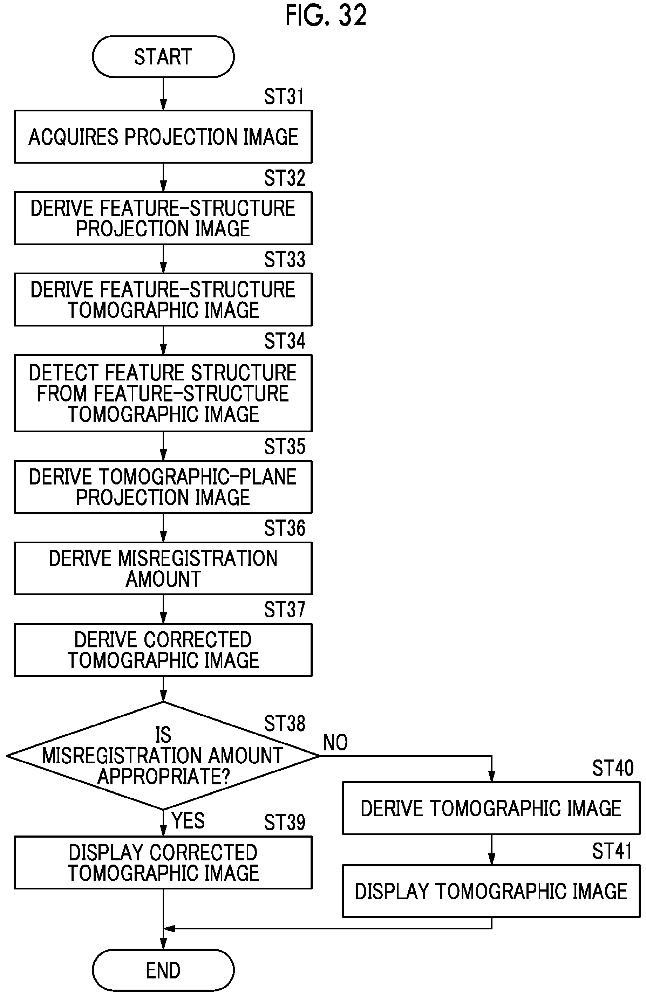

Next, the processing performed in the sixth embodiment will be described. Fig. 32 is a flowchart illustrating processing performed in the sixth embodiment. In Fig. 32, the processing from step ST31 to step ST37 is the same as the processing from step ST1 to step ST7 illustrated in Fig. 18, and thus detailed description thereof will be omitted here. In a case where the reconstruction unit 33 derives the corrected tomographic image Dhj, the misregistration amount determination unit 39 performs image quality evaluation for a region of interest including the feature structure in the corrected tomographic image Dhj, and determines whether or not the derived misregistration amount is appropriate based on a result of the image quality evaluation (step ST38).

In a case where the misregistration amount is appropriate, the display control unit 37 displays the corrected tomographic images Dhj on the display 24 (step ST39), and the processing is ended. The corrected tomographic image Dhj that is derived is transmitted to the image storage system 3, and is stored in the image storage system 3. On the other hand, in a case where the misregistration amount is inappropriate, the reconstruction unit 33 derives the tomographic images Dj by reconstructing the plurality of projection images Gi without correcting the misregistration amount (step ST40). In addition, the display control unit 37 displays the tomographic images Dj on the display 24 (step ST41), and the processing is ended. In this case, the tomographic image Dj is transmitted to the image storage system 3, and is stored in the image storage system 3.

Here, in a case where the misregistration amount is derived by the misregistration amount derivation unit 36, an appropriate misregistration amount may not be derived due to the influence of the structure other than the feature structure. In the sixth embodiment, the image quality evaluation is performed on the corrected tomographic image Dhj, and the determination as to whether the misregistration amount is appropriate or inappropriate is performed based on the result of the image quality evaluation. Therefore, it is possible to appropriately determine whether the derived misregistration amount is appropriate or inappropriate. Further, in a case where it is determined that the misregistration amount is inappropriate, the tomographic image Dj before correction is displayed or stored. Thus, it is possible to reduce a possibility of performing an erroneous diagnosis due to the corrected tomographic image Dhj derived based on the inappropriate misregistration amount.

In the sixth embodiment, the image quality evaluation is performed based on the magnitude of the high-frequency components of the region of interest that is set in the corrected tomographic image Dhj. On the other hand, the present disclosure is not limited thereto. The reconstruction unit 33 may derive a plurality of tomographic images Dj by reconstructing a plurality of projection images Gi without performing the misregistration correction. The misregistration amount determination unit 39 may further perform the image quality evaluation of the region of interest including the feature structure in the tomographic image Dj, compare the result of the image quality evaluation for the corrected tomographic image Dhj and the result of the image quality evaluation for the tomographic image Dj, and determine the tomographic image having higher image quality as the final tomographic image. Here, the final tomographic image is a tomographic image displayed on the display 24 or transmitted to an external device and stored.

Even in the fifth embodiment and the sixth embodiment, the update of the misregistration amount may be repeated as in the fourth embodiment.

Further, also in the fifth embodiment and the sixth embodiment, the misregistration amount derived by the misregistration amount derivation unit 36 may be compared with a predetermined threshold value, and only in a case where the misregistration amount exceeds the threshold value, the tomographic image may be reconstructed while correcting the misregistration amount.

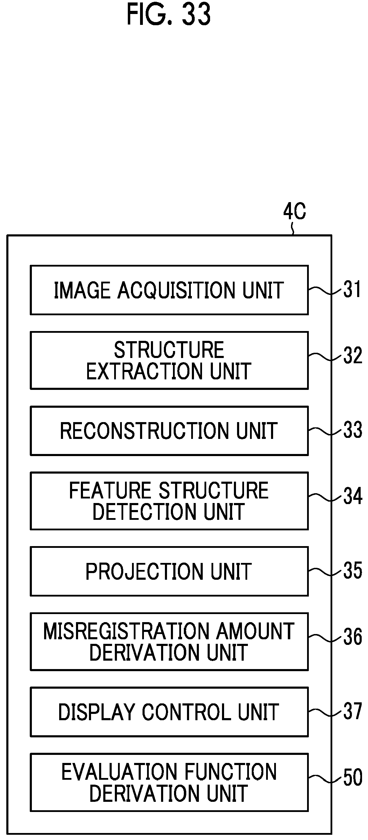

Next, a seventh embodiment of the present disclosure will be described. Fig. 33 is a diagram illustrating a functional configuration of the image processing apparatus according to a seventh embodiment. In Fig. 33, the same reference numerals as those in Fig. 4 are assigned to the same configurations as those in Fig. 4, and detailed description thereof will be omitted here. The seventh embodiment is different from the first embodiment in that the image processing apparatus 4C further comprises an evaluation function derivation unit 50 that derives an evaluation function for performing image quality evaluation for a region of interest including the feature structure in the corrected tomographic image Dhj, and that the misregistration amount derivation unit 36 derives the misregistration amount for optimizing the evaluation function. The processing according to the seventh embodiment can be applied to the second to fifth embodiments, but only a case where the processing is applied to the first embodiment will be described here.

In the seventh embodiment, the evaluation function derivation unit 50 derives a high-frequency image for the region of interest corresponding to the feature structure F, the region of interest being set with respect to the tomographic-plane projection image GTi by the misregistration amount derivation unit 36. The derivation of the high-frequency image may be performed, as in the misregistration amount determination unit 39 according to the sixth embodiment, by performing filtering processing using a Laplacian filter and deriving a secondary differential image. It is assumed that the pixel value of the derived high-frequency image in the region of interest is qkl. k represents a k-th projection image, and 1 represents the number of pixels in the region of interest.

Here, it is assumed that a transformation matrix for correcting the misregistration amount is Wk and a transformation parameter in the transformation matrix is $\theta$k. The transformation parameter $\theta$k corresponds to the misregistration amount. In this case, the image quality evaluation value of the region of interest corresponding to the feature structure F in the corrected tomographic image Dhj can be regarded as an added value of the magnitudes of the high-frequency image of the region of interest after misregistration correction in each of the projection images Gi. By deriving the transformation parameter $\theta$k, that is, the misregistration amount such that the added value is the maximum, the corrected tomographic image Dhj in which the misregistration amount is appropriately corrected can be derived.

Therefore, the evaluation function derivation unit 50 derives the evaluation function shown in the following Expression (3). The evaluation function Ec shown in Expression (3) is an evaluation function Ec to obtain the transformation parameter $\theta$k for minimizing the value in parentheses on the right side with a minus in order to maximize the above addition result. The evaluation function shown in Expression (3) has a plurality of local solutions. Therefore, a constraint condition is applied to the range and the average value of the transformation parameter $\theta$k. For example, a constraint condition is applied such that the average of the transformation parameters $\theta$k for all of the projection images is 0. More specifically, in a case where the transformation parameter $\theta$k is a movement vector representing parallel movement, a constraint condition is applied such that the average value of the movement vectors for all of the projection images Gi is set to 0. In addition, in the seventh embodiment, the misregistration amount derivation unit 36 derives the transformation parameter $\theta$k to minimize the evaluation function Ec shown in the following Expression (3), that is, the misregistration amount.

$$Ec = \arg \min_{\theta} \left( - \sum_k \sum_{l \in ROI} (Wk(\theta k)qkl)^2 \right) \quad (3)$$

As described above, in the seventh embodiment, the image processing apparatus further comprises the evaluation function derivation unit 50 that derives an evaluation function for performing image quality evaluation for a region of interest including the feature structure in the corrected tomographic image Dhj, and the misregistration amount derivation unit 36 derives the misregistration amount for optimizing the evaluation function. Therefore, it is possible to reduce a possibility of performing an erroneous diagnosis due to the corrected tomographic image Dhj derived based on the inappropriate misregistration amount.

In the above embodiments, in order to easily derive the misregistration amount and the temporary misregistration amount, the regions of interest are set in the tomographic image Dj and the tomographic-plane projection image GTi, and the movement direction and the movement amount of the region of interest are derived as the shift vectors, that is, the misregistration amount and the temporary misregistration amount. On the other hand, the present disclosure is not limited thereto. The misregistration amount may be derived without setting the region of interest.

Further, in the above embodiments, the tomographic-plane projection image GTi is derived by the projection unit 35, and the misregistration amount between the tomographic-plane projection images GTi is derived by the misregistration amount derivation unit 36. On the other hand, the present disclosure is limited to thereto. The misregistration amount between the projection images Gi may be derived without deriving the tomographic-plane projection image GTi. In this case, the projection unit 35 is unnecessary in the above embodiments. Further, the misregistration amount derivation unit 36 may derive the misregistration amount based on the positional relationship of the projection images Gi in the corresponding tomographic plane corresponding to the tomographic image from which the feature structure F is detected.

Further, in the embodiments described above, the subject is the breast M, but the present disclosure is not limited thereto. It is needless to say that any part such as the chest or the abdomen of the human body may be the subject.

In the embodiments described above, for example, the following various processors can be used as the hardware structures of processing units that execute various kinds of processing, such as the image acquisition unit 31, the structure extraction unit 32, the reconstruction unit 33, the feature structure detection unit 34, the projection unit 35, the misregistration amount derivation unit 36, the display control unit 37, the focal plane determination unit 38, the misregistration amount determination unit 39, and the evaluation function derivation unit 50. The various processors include, as described above, a CPU, which is a general-purpose processor that functions as various processing units by executing software (program), and a dedicated electric circuit, which is a processor having a circuit configuration specifically designed to execute a specific processing, such as a programmable logic device (PLD) or an application specific integrated circuit (ASIC) that is a processor of which the circuit configuration may be changed after manufacturing such as a field programmable gate array (FPGA).

One processing unit may be configured by one of these various processors, or may be configured by a combination of two or more processors having the same type or different types (for example, a combination of a plurality of FPGAs or a combination of a CPU and an FPGA). In addition, a plurality of processing units may be configured by one processor.

As an example in which the plurality of processing units are configured by one processor, firstly, as represented by a computer such as a client and a server, a form in which one processor is configured by a combination of one or more CPUs and software and the processor functions as the plurality of processing units may be adopted. Secondly, as represented by a system on chip (SoC) or the like, a form in which a processor that realizes the function of the entire system including the plurality of processing units by one integrated circuit (IC) chip is used may be adopted. As described above, the various processing units are configured by using one or more various processors as a hardware structure.

Further, as the hardware structure of the various processors, more specifically, an electric circuit (circuitry) in which circuit elements such as semiconductor elements are combined may be used.

Explanation of References

1: radiography apparatus
2: console
3: image storage system
4, 4A, 4B, 4C: image processing apparatus
10: imaging unit
11: rotation shaft
12: arm part
13: imaging table
14: radiation irradiation unit
15: radiation detector
15A: detection surface
16: X-ray source
17: compression plate
18: support portion
19: moving mechanism
21: CPU
22: image processing program
23: storage
24: display
25: input device
26: memory
27: network I/F
28: bus
31: image acquisition unit
32: structure extraction unit
33: reconstruction unit
34: feature structure detection unit
35: projection unit
36: misregistration amount derivation unit
37: display control unit

38: focal plane determination unit
39: misregistration amount determination unit
40: display screen
41, 42: label
45: warning display
48: structure
50: evaluation function derivation unit
C1 to C4: corresponding point
Dj: tomographic image
Dhj: corrected tomographic image
F1, F3, F4, F5: feature structure
F2: image of feature structure
Gi (i = 1 to n): projection image
Gc: reference projection image
GTi (i = 1 to n): tomographic-plane projection image
H1, H2: search range
M: breast
O1 to O4: pixel position on tomographic plane
P1 to P3: pixel position of projection image
Rf0, R1 to R3, Rh1, Rh2: region of interest
Ra0: peripheral region
Si (i = 1 to n): radiation source position
Sc: reference radiation source position
SDj, SDk: feature-structure tomographic image
SGi (i = 1 to n): feature-structure projection image
Tj: tomographic plane
V10, Vf3: shift vector
X0: optical axis

## Claims

1. An image processing apparatus comprising:

   at least one processor,
   wherein the at least one processor is configured to:

   acquire (ST1) a plurality of projection images that are generated by performing, by an imaging apparatus, tomosynthesis imaging by relatively moving a radiation source with respect to a detection surface of a detection unit and irradiating a subject with radiation at a plurality of radiation source positions due to movement of the radiation source, the plurality of projection images corresponding to the plurality of radiation source positions;
   **characterized in that** the at least one processor is further configured to:

   derive a plurality of feature-structure projection images by extracting a specific structure from the plurality of projection images;
   derive (ST3) a plurality of feature-structure tomographic images respectively for a plurality of tomographic planes of the subject by reconstructing the plurality of feature-structure projection images;
   detect (ST4) at least one feature structure from the plurality of feature-structure tomographic images; and
   derive (ST7) a corrected tomographic image for at least one tomographic plane of the subject by correcting misregistration between the plurality of projection images due to a body movement of the subject by using, as a reference, the feature structure in a corresponding tomographic plane corresponding to the feature-structure tomographic image from which the feature structure is detected, and reconstructing the plurality of projection images.

2. The image processing apparatus according to claim 1,
   wherein the specific structure is at least one of a line structure or a point structure.

3. The image processing apparatus according to claim 2,
wherein the at least one processor is configured to extract at least one of the line structure or the point structure based on a concentration degree of a gradient vector representing a gradient of pixel values in the projection image.

4. The image processing apparatus according to any one of claims 1 to 3,
wherein the at least one processor is configured to:

derive, in the corresponding tomographic plane, a misregistration amount between the plurality of projection images due to the body movement of the subject by using, as a reference, the feature structure; and
derive the corrected tomographic image by correcting the misregistration amount and reconstructing the plurality of projection images.

5. The image processing apparatus according to claim 4,
wherein the at least one processor is configured to:

detect a plurality of feature structures from the plurality of the feature-structure tomographic images;
determine whether or not the corresponding tomographic plane corresponding to the feature-structure tomographic image from which each of the plurality of feature structures is detected is a focal plane; and
derive the misregistration amount in the corresponding tomographic plane determined as the focal plane.

6. The image processing apparatus according to claim 5,
wherein the at least one processor is configured to detect, as the feature structure, a point at which a specific threshold value condition is satisfied in the feature-structure tomographic image.

7. The image processing apparatus according to claim 6,
wherein the at least one processor is configured to:

update the feature-structure tomographic image by reconstructing the feature-structure projection images while correcting the misregistration;
detect an updated feature structure from the updated feature-structure tomographic image;
update the misregistration amount using the updated feature structure; and
repeat the update of the feature-structure tomographic image, the update of the feature structure, and the update of the misregistration amount.

8. The image processing apparatus according to claim 6,
wherein the at least one processor is configured to:

update the feature-structure tomographic image by reconstructing the feature-structure projection images while correcting the misregistration;
detect an updated feature structure from the updated feature-structure tomographic image based on an updated threshold value condition;
update the misregistration amount by using the updated feature structure; and
repeat the update of the feature-structure tomographic image, the update of the feature structure based on the updated threshold value condition, and the update of the misregistration amount.

9. The image processing apparatus according to any one of claims 4 to 8,
wherein the at least one processor is configured to:

derive a tomographic-plane projection image corresponding to each of the plurality of projection images by projecting the plurality of projection images onto the corresponding tomographic plane based on a positional relationship between the radiation source position and the detection unit when performing imaging for each of the plurality of projection images; and
derive, in the corresponding tomographic plane, as the misregistration amount between the plurality of projection images, a misregistration amount between a plurality of the tomographic-plane projection images based on the body movement of the subject, by using, as a reference, the feature structure.

10. The image processing apparatus according to claim 9,
wherein the at least one processor is configured to: set a local region corresponding to the feature structure in the plurality of tomographic-plane projection images; and derive the misregistration amount based on the local region.

11. The image processing apparatus according to claim 9,
wherein the at least one processor is configured to: set a plurality of first local regions including the feature structure in the plurality of tomographic-plane projection images; set a second local region including the feature structure in a tomographic image from which the feature structure is detected; derive misregistration amounts of the plurality of first local regions with respect to the second local region, as temporary misregistration amounts; and derive the misregistration amount based on a plurality of the temporary misregistration amounts.

12. The image processing apparatus according to claim 11,
wherein the at least one processor is configured to derive the temporary misregistration amounts based on a region around the feature structure in the second local region.

13. The image processing apparatus according to claim 11 or 12,
wherein the at least one processor is configured to:

derive a plurality of the tomographic images as target tomographic images by reconstructing the plurality of projection images excluding a target projection image corresponding to a target tomographic-plane projection image that is a target for deriving the misregistration amount; and
derive the misregistration amount for the target tomographic-plane projection image by using the target tomographic image.

14. The image processing apparatus according to any one of claims 4 to 13,
wherein the at least one processor is configured to: perform image quality evaluation of a region of interest including the feature structure in the corrected tomographic image; and determine whether the derived misregistration amount is appropriate or inappropriate based on a result of the image quality evaluation.

15. The image processing apparatus according to claim 14,
wherein the at least one processor is configured to:

derive a plurality of tomographic images by reconstructing the plurality of projection images; and
perform image quality evaluation of a region of interest including the feature structure in the tomographic image; compare the result of the image quality evaluation for the corrected tomographic image with a result of the image quality evaluation for the tomographic image; and determine a tomographic image of which the result of the image quality evaluation is better as a final tomographic image.

16. The image processing apparatus according to any one of claims 4 to 15,
wherein the at least one processor is configured to:

derive an evaluation function for performing image quality evaluation of a region of interest including the feature structure in the corrected tomographic image; and
derive the misregistration amount for optimizing the evaluation function.

17. The image processing apparatus according to any one of claims 1 to 16,
wherein the subject is a breast.

18. The image processing apparatus according to claim 17,
wherein the at least one processor is configured to change a search range in derivation of a misregistration amount according to at least one of a density of a mammary gland, a size of the breast, an imaging time of the tomosynthesis imaging, a compression pressure of the breast in the tomosynthesis imaging, or an imaging direction of the breast.

19. A computer-implemented image processing method comprising:

acquiring (ST1) a plurality of projection images that are generated by performing, by an imaging apparatus, tomosynthesis imaging by relatively moving a radiation source with respect to a detection surface of a detection unit and irradiating a subject with radiation at a plurality of radiation source positions due to movement of the radiation source, the plurality of projection images corresponding to the plurality of radiation source positions; **characterized in that** the method further comprises:

deriving (ST2) a plurality of feature-structure projection images by extracting a specific structure from the plurality of projection images;

deriving (ST3) a plurality of feature-structure tomographic images respectively for a plurality of tomographic planes of the subject by reconstructing the plurality of feature-structure projection images;

detecting (ST4) at least one feature structure from the plurality of feature-structure tomographic images; and

deriving (ST7) a corrected tomographic image for at least one tomographic plane of the subject by correcting misregistration between the plurality of projection images due to a body movement of the subject by using, as a reference, the feature structure in a corresponding tomographic plane corresponding to the feature-structure tomographic image from which the feature structure is detected, and reconstructing the plurality of projection images.

20. A non-transitory storage medium storing a program causing a computer to execute an image processing, the image processing comprising:

acquiring (ST1) a plurality of projection images that are generated by performing, by an imaging apparatus, tomosynthesis imaging by relatively moving a radiation source with respect to a detection surface of a detection unit and irradiating a subject with radiation at a plurality of radiation source positions due to movement of the radiation source, the plurality of projection images corresponding to the plurality of radiation source positions; **characterized in that** the image processing further comprises:

deriving (ST2) a plurality of feature-structure projection images by extracting a specific structure from the plurality of projection images;

deriving (ST3) a plurality of feature-structure tomographic images respectively for a plurality of tomographic planes of the subject by reconstructing the plurality of feature-structure projection images;

detecting (ST4) at least one feature structure from the plurality of feature-structure tomographic images; and

deriving (ST7) a corrected tomographic image for at least one tomographic plane of the subject by correcting misregistration between the plurality of projection images due to a body movement of the subject by using, as a reference, the feature structure in a corresponding tomographic plane corresponding to the feature-structure tomographic image from which the feature structure is detected, and reconstructing the plurality of projection images.

## Patentansprüche

1. Bildverarbeitungsvorrichtung, umfassend:

mindestens einen Prozessor,
wobei der mindestens eine Prozessor so konfiguriert ist, dass er:

mehrere Projektionsbilder erfasst (ST1), die durch Durchführen von Tomosynthesebildgebung durch eine Bildgebungsvorrichtung durch Relativbewegen einer Strahlungsquelle in Bezug auf eine Detektionsfläche einer Detektionseinheit und Bestrahlen einer Untersuchungsperson mit Strahlung an mehreren Strahlungsquellenpositionen aufgrund von Bewegung der Strahlungsquelle erzeugt werden, wobei die mehreren Projektionsbilder den mehreren Strahlungsquellenpositionen entsprechen;
**dadurch gekennzeichnet, dass** der mindestens eine Prozessor ferner so konfiguriert ist, dass er:

mehrere Merkmalsstruktur-Projektionsbilder durch Extrahieren einer spezifischen Struktur aus den mehreren Projektionsbildern ableitet (ST2);
mehrere Merkmalsstruktur-Tomographiebilder jeweils für mehrere Tomographieebenen der Untersuchungsperson durch Rekonstruieren der mehreren Merkmalsstruktur-Projektionsbilder ableitet (ST3);
mindestens eine Merkmalsstruktur aus den mehreren Merkmalsstruktur-Tomographiebildern detektiert (ST4); und
ein korrigiertes Tomographiebild für mindestens eine Tomographieebene der Untersuchungsperson durch Korrigieren von Fehlanpassung zwischen den mehreren Projektionsbildern aufgrund einer Körperbewegung der Untersuchungsperson durch Verwenden der Merkmalsstruktur in einer entsprechenden Tomographieebene, die dem Merkmalsstruktur-Tomographiebild entspricht, aus dem die Merkmalsstruktur detektiert wird, als eine Referenz und durch Rekonstruieren der mehreren Projektionsbilder ableitet (ST7).

**2.** Bildverarbeitungsvorrichtung nach Anspruch 1,
wobei die spezifische Struktur mindestens eine von einer Linienstruktur und einer Punktstruktur ist.

**3.** Bildverarbeitungsvorrichtung nach Anspruch 2,
wobei der mindestens eine Prozessor so konfiguriert ist, dass er mindestens eine der Linienstruktur und der Punktstruktur auf der Grundlage eines Konzentrationsgrads eines Gradientenvektors, der einen Gradienten von Pixelwerten in dem Projektionsbild darstellt, extrahiert.

**4.** Bildverarbeitungsvorrichtung nach einem der Ansprüche 1 bis 3,
wobei der mindestens eine Prozessor so konfiguriert ist, dass er:

in der entsprechenden Tomographieebene einen Fehlanpassungsbetrag zwischen den mehreren Projektionsbildern aufgrund der Körperbewegung der Untersuchungsperson durch Verwenden der Merkmalsstruktur als eine Referenz ableitet; und
das korrigierte Tomographiebild durch Korrigieren des Fehlanpassungsbetrags und durch Rekonstruieren der mehreren Projektionsbilder ableitet.

**5.** Bildverarbeitungsvorrichtung nach Anspruch 4,
wobei der mindestens eine Prozessor so konfiguriert ist, dass er:

mehrere Merkmalsstrukturen aus den mehreren Merkmalsstruktur-Tomographiebildern detektiert;
bestimmt, ob die entsprechende Tomographieebene, die dem Merkmalsstruktur-Tomographiebild entspricht, aus dem jede der mehreren Merkmalsstrukturen detektiert wird, eine Brennebene ist oder nicht; und
den Fehlanpassungsbetrag in der entsprechenden Tomographieebene, die als die Brennebene bestimmt wird, ableitet.

**6.** Bildverarbeitungsvorrichtung nach Anspruch 5,
wobei der mindestens eine Prozessor so konfiguriert ist, dass er als die Merkmalsstruktur einen Punkt detektiert, an dem eine spezifische Schwellenwertbedingung in dem Merkmalsstruktur-Tomographiebild erfüllt ist.

**7.** Bildverarbeitungsvorrichtung nach Anspruch 6,
wobei der mindestens eine Prozessor so konfiguriert ist, dass er:

das Merkmalsstruktur-Tomographiebild durch Rekonstruieren der Merkmalsstruktur-Projektionsbilder aktualisiert, während die Fehlanpassung korrigiert wird;
eine aktualisierte Merkmalsstruktur aus dem aktualisierten Merkmalsstruktur-Tomographiebild detektiert;
den Fehlanpassungsbetrag unter Verwendung der aktualisierten Merkmalsstruktur aktualisiert; und
die Aktualisierung des Merkmalsstruktur-Tomographiebildes, die Aktualisierung der Merkmalsstruktur und die Aktualisierung des Fehlanpassungsbetrags wiederholt.

**8.** Bildverarbeitungsvorrichtung nach Anspruch 6,
wobei der mindestens eine Prozessor so konfiguriert ist, dass er:

das Merkmalsstruktur-Tomographiebild durch Rekonstruieren der Merkmalsstruktur-Projektionsbilder aktualisiert, während die Fehlanpassung korrigiert wird;
eine aktualisierte Merkmalsstruktur aus dem aktualisierten Merkmalsstruktur-Tomographiebild auf der Grundlage einer aktualisierten Schwellenwertbedingung detektiert;
den Fehlanpassungsbetrag unter Verwendung der aktualisierten Merkmalsstruktur aktualisiert; und
die Aktualisierung des Merkmalsstruktur-Tomographiebildes, die Aktualisierung der Merkmalsstruktur auf der Grundlage der aktualisierten Schwellenwertbedingung und die Aktualisierung des Fehlanpassungsbetrags wiederholt.

**9.** Bildverarbeitungsvorrichtung nach einem der Ansprüche 4 bis 8,
wobei der mindestens eine Prozessor so konfiguriert ist, dass er:

ein Tomographieebenen-Projektionsbild, das jedem der mehreren Projektionsbilder entspricht, durch Projizieren der mehreren Projektionsbilder auf die entsprechende Tomographieebene auf der Grundlage einer Positionsbeziehung zwischen der Strahlungsquellenposition und der Detektionseinheit beim Durchführen von Bildgebung für jedes der mehreren Projektionsbilder ableitet; und

in der entsprechenden Tomographieebene als den Fehlanpassungsbetrag zwischen den mehreren Projektionsbildern einen Fehlanpassungsbetrag zwischen mehreren der Tomographieebenen-Projektionsbilder auf der Grundlage der Körperbewegung der Untersuchungsperson ableitet, indem er die Merkmalsstruktur als eine Referenz verwendet.

10. Bildverarbeitungsvorrichtung nach Anspruch 9,
wobei der mindestens eine Prozessor so konfiguriert ist, dass er:

einen lokalen Bereich, der der Merkmalsstruktur entspricht, in den mehreren Tomographieebenen-Projektionsbildern einstellt; und
den Fehlanpassungsbetrag auf der Grundlage des lokalen Bereichs ableitet.

11. Bildverarbeitungsvorrichtung nach Anspruch 9,
wobei der mindestens eine Prozessor so konfiguriert ist, dass er:

mehrere erste lokale Bereiche, die die Merkmalsstruktur enthalten, in den mehreren Tomographieebenen-Projektionsbildern einstellt;
einen zweiten lokalen Bereich, der die Merkmalsstruktur enthält, in einem Tomographiebild, aus dem die Merkmalsstruktur detektiert wird, einstellt;
Fehlanpassungsbeträge der mehreren ersten lokalen Bereiche in Bezug auf den zweiten lokalen Bereich als temporäre Fehlanpassungsbeträge ableitet; und
den Fehlanpassungsbetrag auf der Grundlage von mehreren der temporären Fehlanpassungsbeträge ableitet.

12. Bildverarbeitungsvorrichtung nach Anspruch 11,
wobei der mindestens eine Prozessor so konfiguriert ist, dass er die temporären Fehlanpassungsbeträge auf der Grundlage eines Bereichs um die Merkmalsstruktur in dem zweiten lokalen Bereich ableitet.

13. Bildverarbeitungsvorrichtung nach Anspruch 11 oder 12,
wobei der mindestens eine Prozessor so konfiguriert ist, dass er:

mehrere der Tomographiebilder als Ziel-Tomographiebilder ableitet, indem er die mehreren Projektionsbilder unter Ausschluss eines Zielprojektionsbildes rekonstruiert, das einem Ziel-Tomographieebenen-Projektionsbild entspricht, das ein Ziel zum Ableiten des Fehlanpassungsbetrags ist; und
den Fehlanpassungsbetrag für das Ziel-Tomographieebenen-Projektionsbild durch Verwenden des Ziel-Tomographiebildes ableitet.

14. Bildverarbeitungsvorrichtung nach einem der Ansprüche 4 bis 13,
wobei der mindestens eine Prozessor so konfiguriert ist, dass er:

Bildqualitätsbewertung eines Bereichs von Interesse, der die Merkmalsstruktur enthält, in dem korrigierten Tomographiebild durchführt; und
auf der Grundlage eines Ergebnisses der Bildqualitätsbewertung bestimmt, ob der abgeleitete Fehlanpassungsbetrag geeignet oder ungeeignet ist.

15. Bildverarbeitungsvorrichtung nach Anspruch 14,
wobei der mindestens eine Prozessor so konfiguriert ist, dass er:

mehrere Tomographiebilder ableitet, indem er die mehreren Projektionsbilder rekonstruiert; und
Bildqualitätsbewertung eines Bereichs von Interesse, der die Merkmalsstruktur enthält, in dem Tomographiebild durchführt;
das Ergebnis der Bildqualitätsbewertung für das korrigierte Tomographiebild mit einem Ergebnis der Bildqualitätsbewertung für das Tomographiebild vergleicht; und
ein Tomographiebild, dessen Ergebnis der Bildqualitätsbewertung besser ist, als ein endgültiges Tomographiebild bestimmt.

16. Bildverarbeitungsvorrichtung nach einem der Ansprüche 4 bis 15,
wobei der mindestens eine Prozessor so konfiguriert ist, dass er:

eine Bewertungsfunktion zum Durchführen von Bildqualitätsbewertung eines Bereichs von Interesse, der die Merkmalsstruktur enthält, in dem korrigierten Tomographiebild ableitet; und
den Fehlanpassungsbetrag zum Optimieren der Bewertungsfunktion ableitet.

17. Bildverarbeitungsvorrichtung nach einem der Ansprüche 1 bis 16,
wobei das Untersuchungsobjekt eine Brust ist.

18. Bildverarbeitungsvorrichtung nach Anspruch 17,
wobei der mindestens eine Prozessor so konfiguriert ist, dass er einen Suchbereich bei Ableitung eines Fehlanpassungsbetrags gemäß mindestens einem von einer Dichte einer Brustdrüse, einer Größe der Brust, einer Bildgebungszeit der Tomosynthesebildgebung, einem Kompressionsdruck der Brust bei der Tomosynthesebildgebung und einer Bildgebungsrichtung der Brust ändert.

19. Computerimplementiertes Bildverarbeitungsverfahren, umfassend:

Erfassen (ST1) von mehreren Projektionsbildern, die durch Durchführen von Tomosynthesebildgebung durch eine Bildgebungsvorrichtung durch Relativbewegen einer Strahlungsquelle in Bezug auf eine Detektionsfläche einer Detektionseinheit und Bestrahlen einer Untersuchungsperson mit Strahlung an mehreren Strahlungsquellenpositionen aufgrund von Bewegung der Strahlungsquelle erzeugt werden, wobei die mehreren Projektionsbilder den mehreren Strahlungsquellenpositionen entsprechen;
**dadurch gekennzeichnet, dass** das Verfahren ferner umfasst:

Ableiten (ST2) von mehreren Merkmalsstruktur-Projektionsbildern durch Extrahieren einer spezifischen Struktur aus den mehreren Projektionsbildern;
Ableiten (ST3) von mehreren Merkmalsstruktur-Tomographiebildern jeweils für mehrere Tomographieebenen der Untersuchungsperson durch Rekonstruieren der mehreren Merkmalsstruktur-Projektionsbilder;
Detektieren (ST4) mindestens einer Merkmalsstruktur aus den mehreren Merkmalsstruktur-Tomographiebildern; und
Ableiten (ST7) eines korrigierten Tomographiebildes für mindestens eine Tomographieebene der Untersuchungsperson durch Korrigieren von Fehlanpassung zwischen den mehreren Projektionsbildern aufgrund einer Körperbewegung der Untersuchungsperson durch Verwenden der Merkmalsstruktur in einer entsprechenden Tomographieebene, die dem Merkmalsstruktur-Tomographiebild entspricht, aus dem die Merkmalsstruktur detektiert wird, als eine Referenz und durch Rekonstruieren der mehreren Projektionsbilder.

20. Nicht flüchtiges Speichermedium, das ein Programm speichert, das einen Computer veranlasst, eine Bildverarbeitung auszuführen, wobei die Bildverarbeitung umfasst:

Erfassen (ST1) von mehreren Projektionsbildern, die durch Durchführen von Tomosynthesebildgebung durch eine Bildgebungsvorrichtung durch Relativbewegen einer Strahlungsquelle in Bezug auf eine Detektionsfläche einer Detektionseinheit und Bestrahlen einer Untersuchungsperson mit Strahlung an mehreren Strahlungsquellenpositionen aufgrund von Bewegung der Strahlungsquelle erzeugt werden, wobei die mehreren Projektionsbilder den mehreren Strahlungsquellenpositionen entsprechen;
**dadurch gekennzeichnet, dass** die Bildverarbeitung ferner umfasst:

Ableiten (ST2) von mehreren Merkmalsstruktur-Projektionsbildern durch Extrahieren einer spezifischen Struktur aus den mehreren Projektionsbildern;
Ableiten (ST3) von mehreren Merkmalsstruktur-Tomographiebildern jeweils für mehrere Tomographieebenen der Untersuchungsperson durch Rekonstruieren der mehreren Merkmalsstruktur-Projektionsbilder;
Detektieren (ST4) mindestens einer Merkmalsstruktur aus den mehreren Merkmalsstruktur-Tomographiebildern; und
Ableiten (ST7) eines korrigierten Tomographiebildes für mindestens eine Tomographieebene der Untersuchungsperson durch Korrigieren von Fehlanpassung zwischen den mehreren Projektionsbildern aufgrund einer Körperbewegung der Untersuchungsperson durch Verwenden der Merkmalsstruktur in einer entsprechenden Tomographieebene, die dem Merkmalsstruktur-Tomographiebild entspricht, aus dem die Merkmalsstruktur detektiert wird, als eine Referenz und durch Rekonstruieren der mehreren Projektionsbilder.

**Revendications**

1. Appareil de traitement d'images comprenant :

   au moins un processeur,
   dans lequel l'au moins un processeur est configuré pour :

   acquérir (ST1) une pluralité d'images de projection qui sont générées en effectuant, par un appareil d'imagerie, une imagerie par tomosynthèse en déplaçant relativement une source de rayonnement par rapport à une surface de détection d'une unité de détection et en irradiant un sujet avec un rayonnement à une pluralité de positions de source de rayonnement en raison au déplacement de la source de rayonnement, la pluralité d'images de projection correspondant à la pluralité de positions de source de rayonnement ; **caractérisé en ce que** l'au moins un processeur est en outre configuré pour :

   dériver (ST2) une pluralité d'images de projection de structure caractéristique en extrayant une structure spécifique de la pluralité d'images de projection ;
   dériver (ST3) une pluralité d'images tomographiques de structure caractéristique respectivement pour une pluralité de plans tomographiques du sujet en reconstruisant la pluralité d'images de projection de structure caractéristique ;
   détecter (ST4) au moins une structure caractéristique à partir de la pluralité d'images tomographiques de structure caractéristique ; et
   dériver (ST7) une image tomographique corrigée pour au moins un plan tomographique du sujet en corrigeant un mauvais alignement entre la pluralité d'images de projection en raison d'un mouvement corporel du sujet en utilisant, comme référence, la structure caractéristique dans un plan tomographique correspondant à l'image tomographique de structure caractéristique à partir de laquelle la structure caractéristique est détectée, et en reconstruisant la pluralité d'images de projection.

2. Appareil de traitement d'images selon la revendication 1,
   dans lequel la structure spécifique est au moins l'une d'une structure de ligne ou d'une structure de point.

3. Appareil de traitement d'images selon la revendication 2,
   dans lequel l'au moins un processeur est configuré pour extraire au moins l'une de la structure de ligne ou de la structure de point sur la base d'un degré de concentration d'un vecteur de gradient représentant un gradient de valeurs de pixels dans l'image de projection.

4. Appareil de traitement d'images selon l'une quelconque des revendications 1 à 3, dans lequel l'au moins un processeur est configuré pour :

   dériver, dans le plan tomographique correspondant, une quantité de mauvais alignement entre la pluralité d'images de projection due au mouvement corporel du sujet en utilisant, comme référence, la structure caractéristique ; et
   dériver l'image tomographique corrigée en corrigeant la quantité de mauvais alignement et en reconstruisant la pluralité d'images de projection.

5. Appareil de traitement d'images selon la revendication 4,
   dans lequel l'au moins un processeur est configuré pour :

   détecter une pluralité de structures caractéristiques à partir de la pluralité d'images tomographiques de structure caractéristique ;
   déterminer si oui ou non le plan tomographique correspondant à l'image tomographique de structure caractéristique à partir de laquelle chacune de la pluralité de structures caractéristiques est détectée est un plan focal ; et
   dériver la quantité de mauvais alignement dans le plan tomographique correspondant déterminé comme le plan focal.

6. Appareil de traitement d'images selon la revendication 5,
   dans lequel l'au moins un processeur est configuré pour détecter, comme la structure caractéristique, un point auquel une condition de valeur seuil spécifique est satisfaite dans l'image tomographique de structure caractéristique.

**7.** Appareil de traitement d'images selon la revendication 6,
dans lequel l'au moins un processeur est configuré pour :

mettre à jour l'image tomographique de structure caractéristique en reconstruisant les images de projection de structure caractéristique tout en corrigeant le mauvais alignement ;
détecter une structure caractéristique mise à jour à partir de l'image tomographique de structure caractéristique mise à jour ;
mettre à jour la quantité de mauvais alignement en utilisant la structure caractéristique mise à jour ; et
répéter la mise à jour de l'image tomographique de structure caractéristique, la mise à jour de la structure caractéristique et la mise à jour de la quantité de mauvais alignement.

**8.** Appareil de traitement d'images selon la revendication 6,
dans lequel l'au moins un processeur est configuré pour :

mettre à jour l'image tomographique de structure caractéristique en reconstruisant les images de projection de structure caractéristique tout en corrigeant le mauvais alignement ;
détecter une structure caractéristique mise à jour à partir de l'image tomographique de structure caractéristique mise à jour sur la base d'une condition de valeur seuil mise à jour ;
mettre à jour la quantité de mauvais alignement en utilisant la structure caractéristique mise à jour ; et
répéter la mise à jour de l'image tomographique de structure caractéristique, la mise à jour de la structure caractéristique sur la base de la condition de valeur seuil mise à jour et la mise à jour de la quantité de mauvais alignement.

**9.** Appareil de traitement d'images selon l'une quelconque des revendications 4 à 8, dans lequel l'au moins un processeur est configuré pour :

dériver une image de projection dans un plan tomographique correspondant à chacune de la pluralité d'images de projection en projetant la pluralité d'images de projection sur le plan tomographique correspondant sur la base d'une relation positionnelle entre la position de source de rayonnement et l'unité de détection lors de l'imagerie pour chacune de la pluralité d'images de projection ; et
dériver, dans le plan tomographique correspondant, comme la quantité de mauvais alignement entre la pluralité d'images de projection, une quantité de mauvais alignement entre une pluralité des images de projection dans un plan tomographique sur la base du mouvement corporel du sujet, en utilisant, comme référence, la structure caractéristique.

**10.** Appareil de traitement d'images selon la revendication 9,
dans lequel l'au moins un processeur est configuré pour :

définir une région locale correspondant à la structure caractéristique dans la pluralité d'images de projection dans un plan tomographique ; et
dériver la quantité de mauvais alignement sur la base de la région locale.

**11.** Appareil de traitement d'images selon la revendication 9,
dans lequel l'au moins un processeur est configuré pour :

définir une pluralité de premières régions locales incluant la structure caractéristique dans la pluralité d'images de projection dans un plan tomographique ;
définir une deuxième région locale incluant la structure caractéristique dans une image tomographique à partir de laquelle la structure caractéristique est détectée ;
dériver des quantités de mauvais alignement de la pluralité de premières régions locales par rapport à la deuxième région locale, en tant que quantités de mauvais alignement temporaires ; et
dériver la quantité de mauvais alignement sur la base d'une pluralité des quantités de mauvais alignement temporaires.

**12.** Appareil de traitement d'images selon la revendication 11,
dans lequel l'au moins un processeur est configuré pour dériver les quantités de mauvais alignement temporaires sur la base d'une région autour de la structure caractéristique dans la deuxième région locale.

**13.** Appareil de traitement d'images selon la revendication 11 ou la revendication 12, dans lequel l'au moins un processeur est configuré pour :

dériver une pluralité des images tomographiques en tant qu'images tomographiques cibles en reconstruisant la pluralité d'images de projection en excluant une image de projection cible correspondant à une image de projection dans un plan tomographique cible qui est une cible pour dériver la quantité de mauvais alignement ; et dériver la quantité de mauvais alignement pour l'image de projection dans un plan tomographique cible en utilisant l'image tomographique cible.

**14.** Appareil de traitement d'images selon l'une quelconque des revendications 4 à 13, dans lequel l'au moins un processeur est configuré pour :

effectuer une évaluation de qualité d'image d'une région d'intérêt incluant la structure caractéristique dans l'image tomographique corrigée ; et déterminer si la quantité de mauvais alignement dérivée est appropriée ou inappropriée sur la base d'un résultat de l'évaluation de qualité d'image.

**15.** Appareil de traitement d'images selon la revendication 14,
dans lequel l'au moins un processeur est configuré pour :

dériver une pluralité d'images tomographiques en reconstruisant la pluralité d'images de projection ; et effectuer une évaluation de qualité d'image d'une région d'intérêt incluant la structure caractéristique dans l'image tomographique ;
comparer le résultat de l'évaluation de qualité d'image pour l'image tomographique corrigée avec un résultat de l'évaluation de qualité d'image pour l'image tomographique ; et
déterminer une image tomographique dont le résultat de l'évaluation de qualité d'image est meilleur en tant qu'image tomographique finale.

**16.** Appareil de traitement d'images selon l'une quelconque des revendications 4 à 15, dans lequel l'au moins un processeur est configuré pour :

dériver une fonction d'évaluation pour effectuer une évaluation de qualité d'image d'une région d'intérêt incluant la structure caractéristique dans l'image tomographique corrigée ; et
dériver la quantité de mauvais alignement pour optimiser la fonction d'évaluation.

**17.** Appareil de traitement d'images selon l'une quelconque des revendications 1 à 16, dans lequel le sujet est un sein.

**18.** Appareil de traitement d'images selon la revendication 17,
dans lequel l'au moins un processeur est configuré pour modifier une plage de recherche dans la dérivation d'une quantité de mauvais alignement en fonction d'au moins l'un d'une densité d'une glande mammaire, d'une taille du sein, d'un temps d'imagerie de l'imagerie par tomosynthèse, d'une pression de compression du sein dans l'imagerie par tomosynthèse ou d'une direction d'imagerie du sein.

**19.** Procédé de traitement d'images mis en œuvre par ordinateur comprenant :

acquérir (ST1) une pluralité d'images de projection qui sont générées en effectuant, par un appareil d'imagerie, une imagerie par tomosynthèse en déplaçant relativement une source de rayonnement par rapport à une surface de détection d'une unité de détection et en irradiant un sujet avec un rayonnement à une pluralité de positions de source de rayonnement en raison du mouvement de la source de rayonnement, la pluralité d'images de projection correspondant à la pluralité de positions de source de rayonnement ;
**caractérisé en ce que** le procédé comprend en outre :

dériver (ST2) une pluralité d'images de projection de structure caractéristique en extrayant une structure spécifique de la pluralité d'images de projection ;
dériver (ST3) une pluralité d'images tomographiques de structure caractéristique respectivement pour une pluralité de plans tomographiques du sujet en reconstruisant la pluralité d'images de projection de structure caractéristique ;
détecter (ST4) au moins une structure caractéristique à partir de la pluralité d'images tomographiques de structure caractéristique ; et

dériver (ST7) une image tomographique corrigée pour au moins un plan tomographique du sujet en corrigeant un mauvais alignement entre la pluralité d'images de projection en raison d'un mouvement corporel du sujet en utilisant, comme référence, la structure caractéristique dans un plan tomographique correspondant à l'image tomographique de structure caractéristique à partir de laquelle la structure caractéristique est détectée, et en reconstruisant la pluralité d'images de projection.

20. Support de stockage non transitoire stockant un programme amenant un ordinateur à exécuter un traitement d'images, le traitement d'images comprenant :

acquérir (ST1) une pluralité d'images de projection qui sont générées en effectuant, par un appareil d'imagerie, une imagerie par tomosynthèse en déplaçant relativement une source de rayonnement par rapport à une surface de détection d'une unité de détection et en irradiant un sujet avec un rayonnement à une pluralité de positions de source de rayonnement en raison du mouvement de la source de rayonnement, la pluralité d'images de projection correspondant à la pluralité de positions de source de rayonnement ;
**caractérisé en ce que** le traitement d'images comprend en outre :

dériver (ST2) une pluralité d'images de projection de structure caractéristique en extrayant une structure spécifique de la pluralité d'images de projection ;
dériver (ST3) une pluralité d'images tomographiques de structure caractéristique respectivement pour une pluralité de plans tomographiques du sujet en reconstruisant la pluralité d'images de projection de structure caractéristique ;
détecter (ST4) au moins une structure caractéristique à partir de la pluralité d'images tomographiques de structure caractéristique ; et
dériver (ST7) une image tomographique corrigée pour au moins un plan tomographique du sujet en corrigeant un mauvais alignement entre la pluralité d'images de projection en raison d'un mouvement corporel du sujet en utilisant, comme référence, la structure caractéristique dans un plan tomographique correspondant à l'image tomographique de structure caractéristique à partir de laquelle la structure caractéristique est détectée, et en reconstruisant la pluralité d'images de projection.

## FIG. 1

IMAGE STORAGE SYSTEM ~3

CONSOLE ~2

IMAGE PROCESSING APPARATUS ~4

## FIG. 2

# FIG. 3

4

| 21 | 26 | 27 |
|---|---|---|
| CPU | MEMORY | NETWORK I/F |

28

23 — STORAGE

IMAGE PROCESSING PROGRAM — 22

DISPLAY

24

INPUT DEVICE

25

# FIG. 4

4

| IMAGE ACQUISITION UNIT | 31 |
| STRUCTURE EXTRACTION UNIT | 32 |
| RECONSTRUCTION UNIT | 33 |
| FEATURE STRUCTURE DETECTION UNIT | 34 |
| PROJECTION UNIT | 35 |
| MISREGISTRATION AMOUNT DERIVATION UNIT | 36 |
| DISPLAY CONTROL UNIT | 37 |

## FIG. 5

## FIG. 6

# FIG. 7

M      SD1,D1

$\vdots$

SDm,Dm

# FIG. 8

E4

F1←E1

E2

E3

E5

SDk

# FIG. 9

Si(sxi,syi,szi)

Tj(tx,ty,tz)

Tj

Gi

Pi(pxi,pyi)

## FIG. 10

O1    O2

Tj(tx,ty,tz)

O3    O4

## FIG. 11

Rf0

F1    Dj

GT1 ————— R1
GT2 ————— R2
GT3 ————— R3

## FIG. 12

R3    GT3        R2    GT2        R1    GT1

## FIG. 13

F2    R3        F2    R2        F2    R1

P3        P2        P1

## FIG. 14

F2    R3        F2    R2        F2    R1

V10  P3            P2            P1

## FIG. 15

R2

H2

H1

## FIG. 16

F

Dj

M

F

# FIG. 17

# FIG. 18

START

ST1

ACQUIRES PROJECTION IMAGE

ST2

DERIVE FEATURE-STRUCTURE
PROJECTION IMAGE

ST3

DERIVE FEATURE-STRUCTURE
TOMOGRAPHIC IMAGE

ST4

DETECT FEATURE STRUCTURE
FROM FEATURE-STRUCTURE
TOMOGRAPHIC IMAGE

ST5

DERIVE TOMOGRAPHIC-PLANE
PROJECTION IMAGE

ST6

DERIVE MISREGISTRATION
AMOUNT

ST7

DERIVE CORRECTED
TOMOGRAPHIC IMAGE

ST8

DISPLAY CORRECTED
TOMOGRAPHIC IMAGE

END

# FIG. 19

RfO

F1

F2    R3

P3

F2    R2

P2

F2    R1

P1

# FIG. 20

RfO

F1

F2    R3

Vf3    P3

F2    R2

P2

F2    R1

P1

# FIG. 21

Rf0      Ra0

F1

# FIG. 22

M

SDj_1

SG1      SG2      SG3      · · · · · · ·      SG14      SG15

SDj_1                                        GT1

Rf0_1                                        R1

Vf1

## FIG. 23

START

ST11
ACQUIRES PROJECTION IMAGE

ST12
DERIVE FEATURE-STRUCTURE PROJECTION IMAGE

ST13
DERIVE FEATURE-STRUCTURE TOMOGRAPHIC IMAGE

ST14
DETECT FEATURE STRUCTURE FROM FEATURE-STRUCTURE TOMOGRAPHIC IMAGE

ST15
DERIVE TOMOGRAPHIC-PLANE PROJECTION IMAGE

ST16
DERIVE MISREGISTRATION AMOUNT

ST17
DOES MISREGISTRATION AMOUNT CONVERGE?

NO

ST18
UPDATE FEATURE-STRUCTURE TOMOGRAPHIC IMAGE

YES

ST19
DERIVE CORRECTED TOMOGRAPHIC IMAGE

ST20
DISPLAY CORRECTED TOMOGRAPHIC IMAGE

END

# FIG. 24

BODY MOVEMENT HAS EXCEEDED
THRESHOLD VALUE. DO YOU WANT
TO CORRECT BODY MOVEMENT?

| YES | NO |

~45

# FIG. 25

4A

| IMAGE ACQUISITION UNIT | 31 |

| STRUCTURE EXTRACTION UNIT | 32 |

| RECONSTRUCTION UNIT | 33 |

| FEATURE STRUCTURE DETECTION UNIT | 34 |

| PROJECTION UNIT | 35 |

| MISREGISTRATION AMOUNT DERIVATION UNIT | 36 |

| DISPLAY CONTROL UNIT | 37 |

| FOCAL PLANE DETERMINATION UNIT | 38 |

## FIG. 26

D1
D2
48
D3
D4
D5
D6
D7
D8
D9

## FIG. 27

C2
C1
SDk
F3
C3
C4

# FIG. 28

# FIG. 29

START

ST21

ACQUIRES PROJECTION IMAGE

ST22

DERIVE FEATURE-STRUCTURE PROJECTION IMAGE

ST23

DERIVE FEATURE-STRUCTURE TOMOGRAPHIC IMAGE

ST24

DETECT FEATURE STRUCTURE FROM FEATURE-STRUCTURE TOMOGRAPHIC IMAGE

ST25

DETERMINE FOCAL PLANE

ST26

DERIVE TOMOGRAPHIC-PLANE PROJECTION IMAGE IN CORRESPONDING TOMOGRAPHIC PLANE DETERMINED AS FOCAL PLANE

ST27

DERIVE MISREGISTRATION AMOUNT

ST28

DERIVE CORRECTED TOMOGRAPHIC IMAGE

ST29

DISPLAY CORRECTED TOMOGRAPHIC IMAGE

END

## FIG. 30

4B

| IMAGE ACQUISITION UNIT | 31 |
| STRUCTURE EXTRACTION UNIT | 32 |
| RECONSTRUCTION UNIT | 33 |
| FEATURE STRUCTURE DETECTION UNIT | 34 |
| PROJECTION UNIT | 35 |
| MISREGISTRATION AMOUNT DERIVATION UNIT | 36 |
| DISPLAY CONTROL UNIT | 37 |
| MISREGISTRATION AMOUNT DETERMINATION UNIT | 39 |

## FIG. 31

Dhj

F4
Rh1

F5    Rh2

# FIG. 32

```
        START
          │
          ▼  ST31
┌──────────────────────┐
│ ACQUIRES PROJECTION  │
│       IMAGE          │
└──────────────────────┘
          │
          ▼  ST32
┌──────────────────────┐
│ DERIVE FEATURE-      │
│ STRUCTURE            │
│ PROJECTION IMAGE     │
└──────────────────────┘
          │
          ▼  ST33
┌──────────────────────┐
│ DERIVE FEATURE-      │
│ STRUCTURE            │
│ TOMOGRAPHIC IMAGE    │
└──────────────────────┘
          │
          ▼  ST34
┌──────────────────────┐
│ DETECT FEATURE       │
│ STRUCTURE            │
│ FROM FEATURE-        │
│ STRUCTURE            │
│ TOMOGRAPHIC IMAGE    │
└──────────────────────┘
          │
          ▼  ST35
┌──────────────────────┐
│ DERIVE TOMOGRAPHIC-  │
│ PLANE                │
│ PROJECTION IMAGE     │
└──────────────────────┘
          │
          ▼  ST36
┌──────────────────────┐
│ DERIVE MISREGISTRATION│
│       AMOUNT         │
└──────────────────────┘
          │
          ▼  ST37
┌──────────────────────┐
│ DERIVE CORRECTED     │
│ TOMOGRAPHIC IMAGE    │
└──────────────────────┘
          │
          ▼  ST38
        IS
   MISREGISTRATION AMOUNT ──NO──┐
       APPROPRIATE?             │
          │                     ▼  ST40
         YES  ST39      ┌──────────────────────┐
┌──────────────────────┐│ DERIVE TOMOGRAPHIC   │
│ DISPLAY CORRECTED    ││      IMAGE           │
│ TOMOGRAPHIC IMAGE    │└──────────────────────┘
└──────────────────────┘          │
          │                       ▼  ST41
          │            ┌──────────────────────┐
          │            │ DISPLAY TOMOGRAPHIC  │
          │            │      IMAGE           │
          │            └──────────────────────┘
          │                       │
          ◄───────────────────────┘
          ▼
        END
```

# FIG. 33

4C

| |
|---|
| IMAGE ACQUISITION UNIT — 31 |
| STRUCTURE EXTRACTION UNIT — 32 |
| RECONSTRUCTION UNIT — 33 |
| FEATURE STRUCTURE DETECTION UNIT — 34 |
| PROJECTION UNIT — 35 |
| MISREGISTRATION AMOUNT DERIVATION UNIT — 36 |
| DISPLAY CONTROL UNIT — 37 |
| EVALUATION FUNCTION DERIVATION UNIT — 50 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020067475 A

### Non-patent literature cited in the description

- **YOSHINAGA et al.** Evaluation Method of Concentration Degree and Convergence Index Filter. *Medical Imaging Technology*, 2001, vol. 19 (3)